# EUROPÄISCHE PATENTSCHRIFT

(11) **EP 1 850 890 B1**
(45) Veröffentlichungstag und Bekanntmachung des Hinweises auf die Patenterteilung: **13.02.2013**
(21) Anmeldenummer: 06705831.3
(22) Anmeldetag: 21.01.2006
(51) Int. Cl.: A61L 27/02

(54) **VERFAHREN ZUR MODIFIZIERUNG EINES SUBSTRATS**
METHOD FOR MODIFYING A SUBSTRATE
PROCEDE POUR MODIFIER UN SUBSTRAT

(30) Priorität: 24.02.2005 DE 102005008434
(43) Veröffentlichungstag der Anmeldung: 07.11.2007
(73) Patentinhaber: Forschungszentrum Jülich GmbH, 52425 Jülich (DE)
(72) Erfinder: BERLIN, Peter, 52441 Linnich (DE); JUNG, Adrian, 64289 Darmstadt (DE); WOLTERS, Bernd, 52428 Jülich (DE)
(86) Internationale Anmeldenummer: PCT/DE2006/000093
(87) Internationale Veröffentlichungsnummer: WO 2006/089499

(56) Entgegenhaltungen:
- US-A- 5 728 751
- US-A1- 2001 041 184
- US-A1- 2003 228 419

## Beschreibung

Die Erfindung betrifft ein Verfahren zur Modifizierung eines Substrats.

Die nanoskalige Oberflächen-Modifizierung von Substratmaterialien mit multifunktionalen und/oder biofunktionalen Eigenschaften ist ein wichtiger Zweig der Nanotechnologie, der nahezu alle Zukunftstechnologien, von der Nanoelektronik mit bioelektronischen Funktionsbausteinen über Biosensoren bis hin zu biokompatiblen Materialien, wie Implantaten oder Wirkstoff-Carriern, berührt.

Aminocellulosederivate (ACD) sind bereits seit 1938 aus der US 2,136,299 als Beschichtungsmaterialien für die verschiedensten Substrate, darunter auch undurchdringliche Substrate wie etwa Metalle, bekannt. Sie weisen Klebeeigenschaften auf und können daher als wasserfester Leim für Furniere, aber auch als Haftvermittler für eine weitere Beschichtung des Substrats, beispielsweise mit Drucktinten und Lacken, verwendet werden.

Aus Berlin et al. (Berlin P., Klemm D., Jung A., Liebegott H., Riesler R., Tiller J., Film-forming aminocellulose derivatives as enzyme-compatible support matrices for biosensor developments. Cellulose 2003, Vol. 10, pp. 343-367) ist bekannt, ein Aminocellulosederivat (ACD) auf einem GlasSubstrat aufzubringen. Die auf diese Weise hergestellten relativ dicken ACD-Filme von etwa 100 bis 200 Nanometer werden regelmäßig zur Bildung von Biochip-Oberflächen mit kovalent immobilisierten Biomolekülen versehen und zum Nachweis komplementärer Strukturen verwendet.

Aus Jung et al. (Jung A., Berlin P., Wolters B., Biomoleculecompatible support structures for biomolecule coupling to physical measuring principle surfaces. IEE Proceedings Nanobiotechnol. 2004, Vol 151, No.3, pp. 87-94) ist eine andere Variante der Filmbildung bekannt. Ausgehend von einem Aminocellulosederivat und von einem durch 3-Merkaptopropionsäure mit Carboxylgruppen funktionalisierten Gold-Substrat ist die Bereitung von Biochip-Oberflächen mit kovalent immobilisiertem Enzymprotein bekannt.

Aus der EP 0 941 740 A2 sind aminofunktionale Polysiloxane als ein weiterer Haftvermittler zwischen einer zunächst nicht biokompatiblen Oberfläche und einem Biomolekül bekannt. Die Oberfläche wird mit dem Polysiloxan beschichtet. Anschließend reagiert das Polysiloxan mit dem Biomolekül und geht so mit ihm eine feste Verbindung ein. Durch geeignete Wahl des Biomoleküls kann die Substratoberfläche in dem Sinne biokompatibel gemacht werden, dass Blut beim Kontakt mit der Oberfläche nicht mehr zur Gerinnung angeregt wird.

Nachteilig werden Filmpartikel mit kovalent immobilisiertem Enzymprotein bei Kontakt der Biochip-Oberflächen mit wässrigen Lösungen von der Substratoberfläche wieder abgelöst.

Aufgabe der Erfindung ist es, fest fixierte multifunktionale bzw. biofunktionale Oberflächenstrukturen bereit zu stellen.

Die Aufgabe wird durch ein Verfahren gemäß Hauptanspruch und durch ein Material gemäß Nebenanspruch gelöst. Vorteilhafte Ausgestaltungen ergeben sich aus den darauf rückbezogenen Patentansprüchen.

Das Verfahren weist die erfindungsgemäßen Schritte auf:

Ein Substrat wird mit mindestens einem Aminocellulosederivat und/oder mindestens einem NH₂-(Organo)polysiloxanderivat in Kontakt gebracht.

Es ist dadurch gekennzeichnet, dass sich ein Substratverbundmaterial aus Substrat und Aminocellulosederivat und/oder Substrat und NH₂-(Organo)polysiloxan ausbildet.

Der Begriff Substratverbundmaterial ist hier synonym zu Verbundwerkstoff. Das hergestellte Substratverbundmaterial weist fest verbundene Materialien auf, deren Oberflächeneigenschaften die der Einzelkomponenten übertreffen.
Mit fest verbunden ist gemeint, dass die Oberflächenstrukturen in Lösungsmitteln unterschiedlichster Elektrolytzusammensetzung beispielsweise auch unter Ultraschallbehandlung nicht gelöst werden können. Auch kovalent immobilisierte Biofunktionsmoleküle werden nicht vom Substratverbundmaterial abgelöst.

Vorteilhaft werden durch das erfindungsgemäße Verfahren eine Vielzahl neuer erfindungsgemäßer Substratverbundmaterialien je nach Bedarf für unterschiedlichste Anwendung bereitgestellt.

Das Substratverbundmaterial wird bei Kontakt des Substrats mit einer Modifizierungslösung durch spontane, adhäsive Selbstorganisation eines darin enthaltenen Aminocellulosederivats bzw. NH₂-(Organo)polysiloxanderivats gebildet. Dabei werden Monolagen aus Aminocellulosen-Polymerketten und/oder NH₂-(Organo)polysiloxanen auf dem Substrat unter Beeinflussung der Substratoberflächenstruktur gebildet.

Das durch das erfindungsgemäße Verfahren hergestellte Substratverbundmaterial weist mindestens zwei unterschiedliche Materialien, das heißt ein Substrat mit Aminocellulosederivat bzw. ein Substrat mit NH₂-(Organo)polysiloxanderivat auf. Es weist abhängig von den beteiligten Komponenten die neuen und vorteilhaften Eigenschaften auf, die die Einzelkomponenten nicht aufweisen.

Es ist denkbar, dass das Substratverbundmaterial drei oder mehr Materialien umfasst, z. B. ein Substrat mit einem Aminocellulosederivat und einem NH₂-(Organo)polysiloxan.

Bei Kontakt des Substrats mit der Modifizierungslösung bildet sich durch die komplementär adhäsiven Elektronenstrukturen der Komponenten und durch die daraus folgende Selbstorganisation eine polymere Monolagen-Interfacestruktur aus. Die Komponenten gehen durch eine gemeinsame Elektronenbandstruktur miteinander einen engen Verbund ein.

Sehr vorteilhaft ist es in der Regel ausreichend, das Substrat mit der Modifizierungslösung durch einfachste Mittel miteinander in Kontakt zu bringen. Hierunter fallen einfaches Schwenken, Tauchen, kurzzeitiges Aufbewahren und so weiter. Anspruchsvollere Verfahren, wie z. B. spin-coating, dip-coating, air-brushing, Mikrokontakt-Drucken können, müssen aber nicht, eingesetzt werden.
Spin coating wird z. B. bei 1 bis 20 Tausend Umdrehungen pro Minute, insbesondere 15 Tausend Umdrehungen pro Minute und einer Rotationsdauer von 3 bis 10 Minuten angewendet.
Beim dip-coating kann das Substrat beispielsweise für 5 bis 60 Sekunden, insbesondere für 30 Sekunden in die Modifizierungslösung getaucht werden.

Die Modifizierungslösung kann in einer weiteren Ausgestaltung der Erfindung mittels eines mikro- oder nanostrukturierten Stempels aus einem Polymer, insbesondere aus Poly(dimethylsiloxan) (PDMS), durch Mikro- oder Nanokontakt-Drucken (*µ*CP) auf das Substrat aufgebracht werden.
Dabei werden vorteilhaft die Oberflächenstrukturen beispielsweise in Form eines nanoskaligen Linienmusters mit anwendungsspezifischen Linienabständen und Linienbreiten auf das Substratmaterial gestempelt.
Der Stempel kann hierzu mit der Modifizierungslösung benetzt und danach für 2 bis 15 Minuten mit der Substratoberfläche in Kontakt gebracht werden. Der Stempel kann vorab durch Schütteln in der Modifizierungslösung für eine Zeitdauer von 1 bis 3 Stunden getränkt worden sein und nach dem Tränken 1 bis 2 Minuten im Argon-Strom belassen werden.

Die Substratverbundmaterial-Oberfläche kann durch NH₂-reaktive Funktionalisierung zur Änderung des Wasser-Kontaktwinkels, das heißt zur Änderung der Hydrophobie/Hydrophilie-Balance und/oder zur kovalenten Kopplung mit (Bio-) Funktionsmolekülen oder Nanopartikeln mittels einer NH₂-reaktiven bifunktionellen Reagenz funktionalisiert bzw. chemisch aktiviert worden sein.

Die NH₂-reaktive Funktionalisierung kann vorteilhaft anwendungsspezifisch ausgewählt werden. Dadurch wird vorteilhaft bewirkt, dass positive oder negative Ladungsverteilungen, pH-, Chelat-, Redox- oder Chromogen-Eigenschaften ganzflächig oder in Form von Strukturmustern auf der Substratverbundmaterial-Oberfläche hergestellt werden.

Es sind für diese Verfahren vorteilhaft auch nicht notwendigerweise hohe Temperaturen oder andere Katalysatoren nötig, sondern die spontane Adhäsion tritt auch bei Raumtemperatur auf.

Im Rahmen der Erfindung wurde überraschend gefunden, dass die spontane Adhäsion auf dem Substrat in der Regel schon nach wenigen Minuten (< 5 Minuten) erfolgt. Das Verfahren sieht daher in der Regel ein kurzes in Kontaktbringen des Substrats mit der Modifizierungs-Lösung vor.

Das Verfahren kann zur ganzflächigen Modifizierung beliebig kleiner Substrat-Abmessungen oder zur Oberflächen-Modifizierung in mikrofluidischen (Sensor-)Systemen oder zur Herstellung mikro- und nanoskaliger Oberflächenstruktur-Muster nach dem Prinzip des Mikrokontakt-Druckens eingesetzt werden.

Die Konzentration der eingesetzten Aminocellulosederivate und NH₂-(Organo)polysiloxane darf nicht zu hoch gewählt werden. Ansonsten werden Aggregate der eingesetzten polymeren Derivate auf der Substratoberfläche abgelagert, die nicht als Substratverbundmaterial im Sinne der Erfindung gelten.

Besonders vorteilhaft wird als Modifizierungslösung eine 0,05 bis 0,5%ige Aminocellulosederivatlösung der Formel I verwendet (s. u.). Es ist denkbar, höhere Konzentrationen bis 5% zu verwenden, allerdings muss dann der Waschvorgang intensiviert werden.

In einer anderen Ausgestaltung der Erfindung wird als Modifizierungslösung eine 0,03 bis 1%igen NH₂-(Organo)polysiloxan-lösung der allgemeinen Formeln P1 bis P5 (s.u.) verwendet. Besonders vorteilhaft ist eine Konzentration von etwa 0,03 bis 0,1%.

Das Substrat wird nach Kontakt mit der Modifizierungslösung mit dem jeweiligen Lösemittel gewaschen, z. B. durch Schütteln bei mehrfacher Lösemittelanwendung oder im Ultraschallbad.
Bei einer auf diese Weise durchgeführten Substratbehandlung liegen die auf dem Substrat angeordneten Polymerketten in einer Dickenabmessung < 1 bis 3 Nanometer vor.
Dies entspricht im Falle eines Aminocellulosederivats etwa nur einer Monolage der entsprechend auf dem Substrat aufgebrachten Polymerketten.

Die Polymerketten sind auf dem Substrat über genannte gemeinsame Elektronenbandstrukturen fest fixiert und verleihen dem gebildeten Substratverbundmaterial eine neue Qualität in Bezug auf eine spätere Anwendung.

Im Rahmen der Erfindung wurde erkannt, dass je nach Art der verwendeten Aminocellulosederivate und/oder NH₂-(Organo)polysiloxane erstmalig ein Strukturdesign auf dem Substrat für die weitere vorzugsweise biophysikalische oder biomedizinische Anwendung möglich ist.

Ein grundlegender Vorteil bei der Verwendung der Polysaccharidstrukturen in Form von Cellulosestrukturen ist, dass Polysaccharide natürlicherseits in Gesellschaft mit Proteinen bzw. Zellen vorkommen und an diese binden.

In einer weiteren Ausgestaltung der Erfindung werden die Substratverbundmaterialien mit Funktionsmolekülen versehen, die je nach Art der Verwendung ausgewählt werden können.
Dadurch wird vorteilhaft bewirkt, dass auf die Monolagen aus Aminocellulosederivat und/oder NH₂-(Organo)polysiloxanen weitere Moleküle, z. B. Bio-Funktionsmoleküle durch elektrostatische oder kovalente Kopplung aufgebracht werden. Diese dienen dann z. B. zum Nachweis eines Analyten mit Komplementärstruktur.
Es ist denkbar, durch ein derartiges Strukturdesign eine Protein- oder Zelladhäsion oder eine Körperabwehrreaktion zu fördern bzw. zu vermeiden.

Die erfindungsgemäßen Substratverbundmaterialien finden beispielsweise Anwendung bei der Herstellung von Biochips und Implantaten mit verbesserten biokompatiblen Oberflächeneigenschaften im Sinne einer verbesserten Körperverträglichkeit. Es können besonders vorteilhaft auch geeignete textile Substrate, z. B. Baumwolle mit gewünschten Modifizierungslösungen strukturiert werden.

Das erfindungsgemäße Verfahren und die Substratverbundmaterialien werden insbesondere zur Entwicklung nanostrukturierter biofunktionaler Implantatoberflächen angewendet.
Dabei kann das erfindungsgemäße Oberflächenstrukturdesign auf alle biomedizinisch relevanten Substratmaterialien bzw. Implantate zur Herstellung der als biokompatibel erkannten Oberflächenstrukturen, wie hydrophobe, hydrophile, elektrostatisch negative, biofunktionalisierte, nanoskalig Strukturgemusterte und Zelladhäsive und/oder topografisch definierte Oberflächen, angewendet werden.

Derartige Eigenschaften und Anwendungsmöglichkeiten bieten nur die erfindungsgemäßen Substratverbundmaterialien, nicht jedoch die Einzelkomponenten und keinesfalls unmodifizierte Substrate bzw. Implantate.

Den Substraten, die mit den Aminocellulosederivaten oder NH₂-(Organo)polysiloxanderivaten Substratverbundmaterialien bilden, ist gemeinsam, dass sie in Bezug auf die Derivate komplementär adhäsive Elektronenstrukturen vorzugsweise durch Sauerstoff- bzw. Hydroxy-Funktionen auf den Substratoberflächen besitzen, die die adhäsive Selbstorganisation der Aminocellulosederivate und/oder NH₂-(Organo)polysiloxane insbesondere über deren NH₂-Gruppen bewirken und somit für den festen Verbund sorgen.

Besonders vorteilhaft ist man bei der Wahl des Substrats nicht eingeschränkt.
Es können biophysikalisch und medizinisch relevante oder auch textile Substrate gewählt werden, sofern diese geeignet sind, mit den Aminocellulosederivaten und/oder NH₂-(Organo) polysiloxanen über spontane Adhäsion ein Substratverbundmaterial mit den oben genannten Eigenschaften auszubilden.

Substrate, die nur geringe bis keine adhäsiven Eigenschaften bei Kontakt mit einer Modifizierungslösung entfalten, werden vorab in einer weiteren Ausgestaltung der Erfindung mit Sauerstoffplasma oder durch ein anderes Sauerstoff- oder OH-Funktionen erzeugendes Verfahren behandelt.

Besonders vorteilhaft werden sie zu diesem Zweck vorab mit einem ultradünnen SiOₓ-Polymerfilm mit einer Dicke < 1 bis 2 Nanometer beschichtet.

Es ist im Rahmen des Verfahrens ohne weiteres denkbar, verschiedenartige Substrate, z. B. zur Bildung eines Array, nebeneinander in einer Ebene anzuordnen.

Als Substrate können eingesetzt werden: Glastyp-Substrate (hydrophilisiert oder pyrolytisch SiOₓ-Polymer-beschichtet), Si- oder SiO₂-Substrate mit nativer oder thermisch erzeugter SiO₂-Polymerschicht oder pyrolytisch mit SiOₓ-Polymer beschichtet und Metall- und Metall/Metalloxid-Substrate. Hierunter fallen z. B. Gold, Silber, Platin, Titan, Tantal, Aluminium, Zirkon, Vanadium, Niob, Chrom, Molybdän, Wolfram, Mangan, Technetium, Rhenium, Ruthenium, Osmium, Kobalt, Rhodium, Iridium, Nickel, Palladium, Kupfer und deren Oxide.

Makromolekulare Substrate, wie Keramik aus z. B. Zirkonoxid oder Nanopartikel, wie Gold-, SiO₂- oder Metalloxid-Nanopartikel sind ebenfalls umfasst.
Weiterhin kommen in Betracht: Polymere mit Hydroxygruppen bzw. Sauerstoff-Funktionen, z. B. Polysaccharide (Cellulosen in Faser- oder Hohlfaser-Form und Bakteriencellulosen in Flächen- oder Röhrenform). Polysiloxane bzw. (Organo)polysiloxane, wie Polydimethylsiloxan (PDMS), NH₂-(Organo)polysiloxane, Polymethylmethacrylat, Poly-N-isopropylacrylamid (PNiPAM), Poly-Glykolid-co-Lactid (PGL).

Polymere mit Carboxyl- oder Sulfogruppen (Polyhydroxiethylmethacrylat (PHEMA), Kationen-bindende Polystyrole), Proteine (Kollagene, Glykoproteine) und textile Substrate, wie Baumwolle, Wolle.

Die multifunktionalen Oberflächenstrukturen der Substratverbundmaterialien sind charakterisiert durch ihre Dickenabmessungen kleiner 1-3 Nanometer.

Sie weisen eine hohe Variabilität in der Hydrophilie- bzw. Hydrophobiebalance auf, gekennzeichnet durch einen Wasser-Kontaktwinkel kleiner 40 bis größer 90 Grad.

Sie weisen weiterhin eine große strukturelle Variabilität der kovalenten Funktionalisierungsmöglichkeiten, ausgehend von NH₂-Ankergruppen-Dichten von 0,2 bis 5 nMol pro cm² der Substratoberfläche, auf.

Eine definierte Oberflächentopografie bei RMS-Rauhigkeiten von 0,5 bis 2 Nanometer durch AFM vermessen ist die Regel. Sofern das Substrat vor der Modifizierung mit einem Plasma, insbesondere mit einem Argon- oder Sauerstoff-Plasma behandelt wird, werden vorteilhaft besonders geringe RMS-Rauhigkeiten kleiner 0,5 Nanometer der Substratverbundmaterialoberfläche gebildet.

Zur Bereitung der Modifizierungslösung werden die Aminocellulosederivate vorzugsweise in bidestilliertem Wasser oder Dimethylacetamid (DMA) gelöst.
NH₂-(Organo)polysiloxane werden vorzugsweise in Methanol, Ethanol oder 2-Propanol gelöst.
Sie werden vorzugsweise mittels Zentrifugalfilterröhrchen mit einer Porenweite von 0,2 bis 0,45 *µ*m filtriert.

### 1. Aminocellulose- und NH₂- (Organo)polysiloxanderivate

Als Aminocellulosederivate kommen z. B. alle im nachfolgend genannten Formelschema 1 Verbindungen in Betracht.

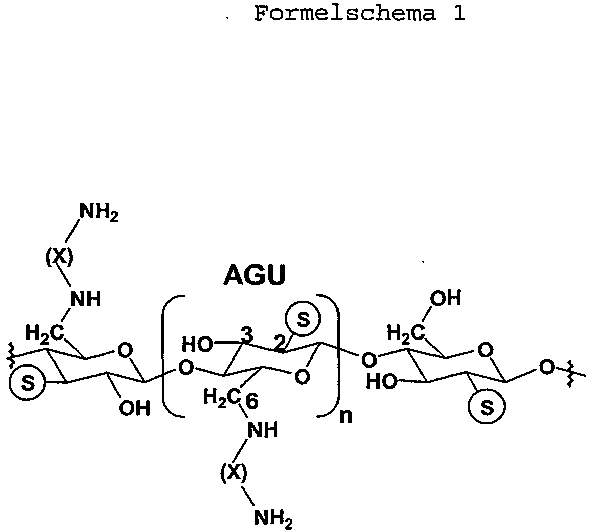

### Allgemeine Formel I: Anhydro-Glucoseeinheit (AGU)

Mögliche Substituenten an der AGU sind:
S = Acetat-, Benzoat-, Carbanilat-, Propionat-, Tosylat- oder Methoxy-Gruppen, gemäß dem Substitutionsgrad von S (DSₛ: 0 < DSₛ < 2 an C2/C3 der AGU).
(X) = Spacergruppen, gemäß dem Substitutionsgrad von -NH (X) NH₂ (DS_{NH}(_{X})_{NH2} 0 < DS_{NH(X)NH2} < 1) an C6 der AGU) : Siehe Typ a bis d in Formelschema 1.
n = 100 bis 1500, vorzugsweise 200.

Derivate der Aminocellulose-Leitstruktur gemäß Allgemeiner Formel I können sein:
Typ a: (X)= Alkylen-Rest (CH₂) _{i;} i= 2, 3, 4, 5, 6, 7, 8, 9, 10, 11 oder 12;
Typ b: (X)= Oligoamin-Rest, z. B.
-CH₂-CH₂-NH-CH₂-CH₂-, ("DETA-Cellulose") oder
-CH₂-CH₂-CH₂-NH-CH₂-CH₂-CH₂-, ("DPTA-Cellulose") oder
-CH₂-CH₂-NH-CH₂-CH₂-NH-CH₂-CH₂-, ("TETA-Cellulose") oder
-CH₂-CH₂-NH-CH₂-CH₂-NH-CH₂-CH₂-NH-CH₂-CH₂-, ("TEPA-Cellulose") als Isomere oder 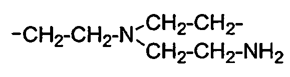 ("TETAT-Cellulose")

Diese Derivate des Typ b weisen vorzugsweise als Substituent S ein Tosylat auf, wobei das Derivat in Wasser löslich ist, oder aber ein Carbanilat auf, wobei das Derivat in DMA löslich ist.

### Typ c: (X)= Aryl oder Aryl-Alkylen-Rest, z. B.:

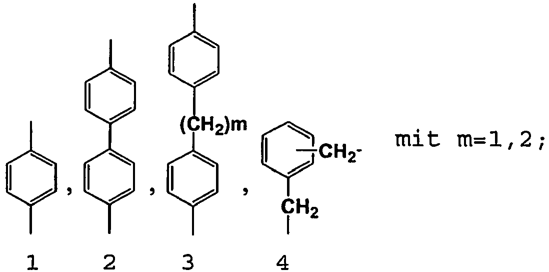

(1) = PDA-Cellulosetosylat oder
(4a)= XDAₒ-Cellulosecarbanilat
(4b)= XDAₘ-Cellulosecarbanilat
(4c)= XDAₚ-Cellulosecarbanilat

### Typ d: N,N-disubstituierte PDA-Cellulosen, mit redoxchromogenen Eigenschaften, z. B.:

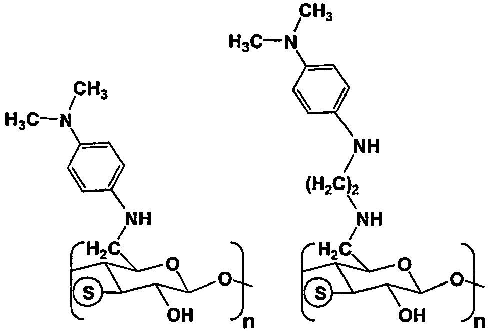

Die oben genannten Derivate gemäß Formelschema 1 können durch weitere Derivatisierungen von Tosylcellulose bzw. Tosylcellulosederivaten mit Diaminen, Oligoaminen bzw. Polyaminen erweitert werden.

Als NH₂-(Organo)polysiloxanderivate kommen z. B. die nachfolgend in Formelschema 2 genannten Verbindungen in Betracht.

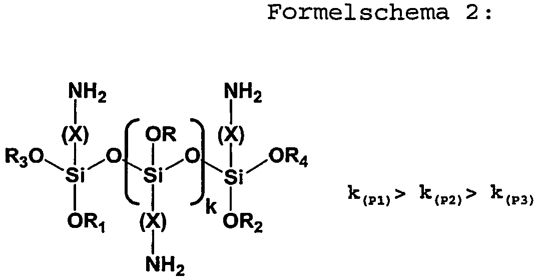

### Allgemeine Formel II

Die NH₂-(Organo)polysiloxanderivate P1 bis P3 werden durch ein Gemisch aus NH₂-(Organo)silan/Wasser, vorzugsweise in den Mol-Verhältnissen 1:3 (P1), 1:2 (P2) und 1:1 (P3), hergestellt.

Für die Reste gelten nachfolgende Vorgaben:
Entweder ist
   R₁ und R₂= H oder Methyl bzw. Ethyl und
   R, R₃ und R₄= NH₂-(Organo)polysiloxan-Strukturen,
oder es ist
   R₁ und R₃= H oder Methyl bzw. Ethyl und
   R, R₂ und R₄= NH₂-(Organo)polysiloxan-Strukturen,
oder es ist
   R₁ und R₄= H oder Methyl bzw. Ethyl und
   R, R₂ und R₃= NH₂-(Organo)polysiloxan-Strukturen,
oder es ist
   R₂ und R₄= H oder Methyl bzw. Ethyl und
   R, R₁ und R₃= NH₂-(Organo)polysiloxan-Strukturen,
oder es ist
   R₂ und R₃= H oder Methyl bzw. Ethyl und
   R, R₁ und R₄= NH₂-(Organo)polysiloxan-Strukturen.

Für die Substituenten (X) in P₁ bis P₃ gilt:

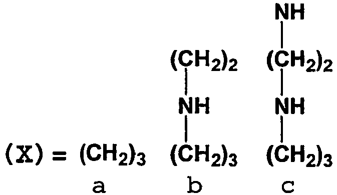

Es können als NH₂-(Organo)polysiloxanderivate aber auch die nachfolgenden Verbindungen P4 bis P5 eingesetzt werden.

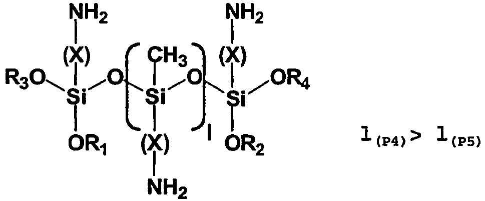

### Allgemeine Formel II (Fortsetzung)

Die NH₂-(Organo)polysiloxanderivate P4 und P5 werden durch ein Gemisch aus NH₂-(Organo)silan/Wasser, vorzugsweise in den Mol-Verhältnissen 1:2(P4) bzw. 1:1 (P5), hergestellt.

Für die Reste gelten nachfolgende Vorgaben:
Entweder ist
   R₁ und R₂= H oder Methyl bzw. Ethyl und
   R₃ und R₄= NH₂-(Organo)polysiloxan-Strukturen,
oder es ist,
   R₁ und R₃= H oder Methyl bzw. Ethyl und
   R₂ und R₄= NH₂-(Organo)polysiloxan-Strukturen,
oder es ist
   R₁ und R₄= H oder Methyl bzw. Ethyl und
   R₂ und R₃= NH₂-(Organo)polysiloxan-Strukturen,
oder es ist
   R₂ und R₄= H oder Methyl bzw. Ethyl und
   R₁ und R₃= NH₂-(Organo)polysiloxan-Strukturen.

Für die Substituenten (X) in P4 bis P5 gilt:

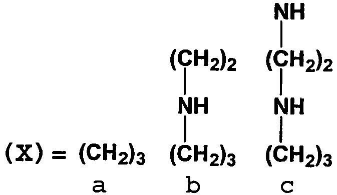

Die oben genannten NH₂-(Organo)polysiloxanderivate gemäß Formelschema 2 können besonders vorteilhaft auch in Kombination mit den Aminocellulosederivaten beim erfindungsgemäßen Strukturdesign der Substratverbundmaterialien eingesetzt werden.

In einer weiteren Ausgestaltung der Erfindung kann das Substrat im Falle des erfindungsgemäßen Strukturdesigns durch ein NH₂-(Organo)polysiloxanderivat vorab mittels des hydrophilen SiOₓ-Polymers in Dickenabmessungen kleiner als 1 bis 2 Nanometer pyrolytisch modifiziert werden.
Dies gelingt vorteilhaft durch eine einfache und kurzzeitige, das heißt in einer weniger als 1 Sekunde andauernden, Behandlung des Substrats mittels Verfahren gemäß 2.3.

Das erfindungsgemäße Verfahren lässt sich dann auch bei solchen Substratmaterialien anwenden, die nicht von vornherein mit den Derivaten gemäß Formelschema 1 und 2 spontan adhäsiv getriebene Oberflächenstrukturen ausbilden.

### 2.1 Oberflächenstrukturdesign von Substraten mit Aminocellulosederivaten

Grundlage der Derivatisierung der Aminocellulosen-Leitstruktur ist die unterschiedliche S_{N2}-Reaktivität der OH-Funktionen an C6 bzw. C2/C3 der AGU, s. Allgemeine Formel I.

Der allgemeine Derivatisierungs-Ansatz geht z. B. von einem 6(2)-O-Tosylcellulosederivat, vorzugsweise von kommerziell erhältlichen 6(2)-O-Tosylcellulosen oder 6(2)-O-Tosylcellulose-carbanilaten aus, die an C6 der AGU einen reaktiven Tosylat-Rest und an C2/C3 der AGU löslichkeitsvermittelnde Substituentengruppen, wie Tosylat oder Carbanilat mit unterschiedlichem Substitutionsgrad DS (0< DSₛ <2) besitzen (s. "S" in der allgemeinen Formel I).

Der Tosylat-Rest an C6 wird in einem Modifizierungsansatz durch Diamin- oder Oligoamin-Verbindungen H₂N-(X)-NH₂" substituiert (s. (X) in Formel I). Dazu wird die Tosylcellulose bzw. das Tosylcellulosecarbanilat in Dimethylsulfoxid (DMSO) mit einem Modifizierungs-Reagenz H₂N-(X)-NH₂ (s. (X), Typ a bis d in Formelschema 1) gemischt und für 3 bis 6 h auf 70 bis 100°C erhitzt. Nach Abkühlung wird das Reaktionsgemisch in ein Gefäß mit Tetrahydrofuran gegossen. Dabei fällt das gewünschte Aminocellulosen-Derivat als Feststoff aus. Das Derivat wird isoliert, mit Tetrahydrofuran und Ethanol gewaschen und danach getrocknet. Das Aminocellulosen-Derivat ist je nach Struktur des Substituenten S (s. allgemeine Formel I) und Substitutionsgrad DSₛ an C2/C3 in Wasser oder Dimethylacetamid (DMA) löslich.

Auf diese Weise werden alle Derivate der Aminocellulose gemäß Formelschema 1 hergestellt.

Das erfindungsgemäße Verfahren stützt sich daher besonders vorteilhaft auf die vielfältigen strukturellen Modifizierungsmöglichkeiten der Aminocellulosen nach dem allgemeinen Derivatisierungs-Ansatz.

Die Derivatvielfalt lässt sich vorteilhaft komplettieren, wenn der allgemeine Derivatisierungsansatz von Tosylcellulosederivaten mit Substituenten-Gruppen S, wie z. B. Acetat, Propionat, Benzoat, Methoxy an C2/C3 der AGU ausgeht und weitere Diamine, Oligoamine oder Polyamine in die Substitutionsreaktion an C6 einbezogen werden.

### 2.1.1 Spacereffekt und strukturelle Eigenschaftsmuster durch (X) an AGU-Position C6

Abgestufte Abstände (Spacereffekt) der NH₂-Endgruppen zur Cellulosekette werden an AGU-Position C6 dadurch bereit gestellt, dass (X) in der allgemeinen Formel I eine Alkylen-, Aryl-, Aralkylen- oder Oligoamin-Struktur ist (s. (X)-Typ a bis d in Formelschema 1). Beispielsweise variieren die Matrixabstände zwischen etwa 0,4 und 2 nm, wenn Derivate mit Strukturen (X) der Typ-Reihe a bzw. b aus Formelschema 1 eingesetzt werden.

Durch Strukturen (X) der Typ-Reihen a bis c werden insbesondere die Reaktivitäts- bzw. spontanen Adhäsions-Eigenschaften entlang der Aminocellulosen-Polymerketten modifiziert, wie auch die pH-Eigenschaften sowie die Hydrophilie- bzw. Hydrophobie-Balance.

Vorteilhaft kann beispielsweise mit zunehmender Alkylen-Kettenlänge (X) gemäß Typ a aus Formelschema 1 das hydrophobe Eigenschaftsmuster der entsprechenden Derivate dominanter und der Spacereffekt größer eingestellt werden.

Sofern besondere Elektronentransfereigenschaften des Substratverbundmaterials erwünscht sind, können Aminocellulosederivate mit EDA- (Typs a, i=2) oder mit Oligoamin-Resten (Typ b) an C6 verwendet werden, da diese Derivate Chelate mit Schwermetall-Ionen, z. B. blaugefärbte Cu²⁺-Chelate(λ_{Max}-Werte = 560 bis 630 nm), ausbilden, die im Falle ihrer Anwendung den entsprechenden Substratoberflächen besondere Elektronentransfereigenschaften verleihen. Deshalb sind sie für die Kopplung mit biologischen Redoxsystemen insbesondere mit Cu-Proteinen von besonderer Bedeutung.

Derivate mit Spacerstrukturen (X) des Typs c und d sind vorteilhaft redoxaktiv bzw. -chromogen. Diese Eigenschaften weisen bei adhäsiver Fixierung auf Substratoberflächen je nach Struktur (X) und redoxchromogener Folgereaktion spezielle Elektronentransfer-Eigenschaften auf.

Der Grad der strukturellen Modifizierung mittels (X) entlang der Aminocellulosenpolymerketten ist durch den Substitutionsgrad DS_{NH(x)NH2} veränderbar.
Dies determiniert bei lateraler Übertragung auf die Substratoberfläche die Dichte der funktionellen Gruppen und in Relation zur Substitution S bzw. DSₛ die funktionelle Eigenschaft auf der Substratoberfläche.

### 2.1.2 Löslichkeit und Hydrophilie- bzw. Hydrophobie-Balance mittels S an AGU-Position C2/C3

Vorteilhafte Eigenschaften erhalten die Aminocellulosederivate auch mittels Substitution der OH-Gruppen an C2/C3 durch unterschiedliche Estergruppen. Dies hat auf die Löslichkeit der Aminocellulosederivate einen determinierenden Einfluss. Der Substitutionsgrad DSₛ, das heißt das Verhältnis von OH-/Ester-Gruppen an den (Aminocellulosen-)Polymerketten entscheidet darüber, ob das Derivat in Wasser oder in einem organischen Lösungsmittel, z. B. DMA löslich ist: Darüber hinaus beeinflusst der DSₛ die Hydrophilie- bzw. Hydrophobie-Balance. Darüber hinaus geht auch von den Strukturen an den AGU-Positionen C2/C3 (OH- bzw. Ester-Gruppen) ein Einfluss auf die adhäsiven Elektronenstruktur-Eigenschaften der Aminocellulosenpolymerketten aus.

Beispielsweise sind EDA-Cellulosetosylate (Typ a, i=2) bzw. Aminocellulosentosylate der (X)-Typ-Reihe b aus Formelschema 1 bei DS_{Tosylat}-Werten von 0,1 bis 0,2 in Wasser löslich. In wässrigem Milieu stellen sich bei diesen Derivaten pH-Werte zwischen 10 und 11 ein.

Für das Strukturdesign lassen sich vorteilhaft durch Titration z. B. mit 5n HCl Biomolekül-relevante pH-Optima, z. B. pH 5,5 bis 8, einstellen.

### 2.2 Oberflächenstrukturdesign von Substraten mittels NH₂-(Organo)polysiloxanderivate

Die NH₂-(Organo)polysiloxane der allgemeinen Formeln P1 bis P5 aus Formelschema 2 werden aus NH₂-(Organo)alkoxysilan/Wassergemischen oder NH₂-(Organo)alkoxysilan/Wasser/Ethanolgemischen oder NH₂-(Organo)alkoxysilan/Wasser/Methanolgemischen oder vorzugsweise NH₂-(Organo)alkoxysilan/Wasser/2-Propanol-Gemischen gebildet. Die Zusammensetzung kann variieren, z.B. zwischen Molverhältnissen (Organo)alko-xysilan/Wasser wie 1:3, 1:2 oder 1:1, und Zusatz einer katalytischen Menge 1n HCl durch 3 bis 4-stündiges Rühren.

Die erhaltenen NH₂-(Organo)polysiloxane lösen sich vorteilhaft zwischen 0,03 und 1%-ig z. B. in Methanol, Ethanol oder 2-Propanol und stehen sodann für die erfindungsgemäße Oberflächen-Modifizierung bereit.

Als (Organo)alkoxysilane werden für die Herstellung der NH₂-(Organo)polysiloxane beispielsweise 3-Aminopropyl-trimethoxysilan, 3-Aminopropyl-triethoxysilan, 3-[2-(2-Amino-ethylamino)ethylamino]propyl-trimethoxysilan, 3-(2-Aminoethylamino)propylmethyl-dimethoxysilan oder vorzugsweise 3-(2-Aminoethylamino)propyl-trimethoxysilan bzw. Silan-Gemische aus NH₂-(Organo)alkoxysilanen oder aus NH₂-(Organo)alkoxysilan und (Organo)alkoxysilan (ohne NH₂-Gruppen) oder aus NH₂-(Organo)alkoxysilan und Tetraalkoxysilan eingesetzt.

Die NH₂-(Organo)polysiloxan-Derivate werden vorzugsweise in Ethanol- oder 2-Propanol- Lösungen angewendet und vor ihrem Einsatz z. B. mittels Zentrifugal-Filter-Röhrchen (Porenweite z. B. 0,2 bis 0,45 *µ*m) filtriert.

Besonders vorteilhaft ist die Anwendung der relativ hydrophoben NH₂-(Organo)polysiloxanderivate in Kombination mit Aminocellulosederivaten.
Vorteilhaft werden beispielsweise Substratverbundmaterialien mit mikro- und nanoskaligen Mustern alternierender hydrophober und NH₂-Gruppenhaltiger Oberflächenstrukturen oder alternierender NH₂-Gruppenhaltiger Oberflächenstrukturen mit unterschiedlichen NH₂-Spacerlängen (X) ausgehend von NH₂-(Organo)polysiloxan-modifizierten Substratoberflächen und *µ*CP mittels entsprechender Aminocellulosederivate hergestellt.

Vorteilhaft ist auch die Anwendung von NH₂-(Organo)polysiloxanderivaten zur Hydrophobisierung von geeigneten textilen Substraten oder beim Oberflächenstrukturdesign von Aluminiumoxid- bzw. Zirkonoxid-Keramiken oder von Biochips, vorzugsweise ausgehend von Glastyp-, Si/SiO₂-, Gold-, PDMS-Substraten, und anschließender Bio-Funktionalisierung, z. B. in mikrofluidischen Sensorsystemen.

Bei anderen kombinierten Anwendungen von NH₂-(Organo)polysiloxanen und Derivaten der Amino-cellulosen-Leitstruktur zur Herstellung von Substratverbundmaterialien wird z. B. folgendermaßen verfahren: Ausgehend von SiOₓ-Polymer-modifizierten Glastyp-Substraten oder von Si/SiO₂-Substraten, wird die Substratoberfläche mittels NH₂-(Organo)polysiloxanderivat P1a gemäß Formelschema 2 modifiziert. Anschließend wird z. B. mittels 0,1 n HCl protoniert und danach beispielsweise mit einer Modifizierungslösung von EDA-Cellulosecarbanilat(Typ a, i=2) gemäß (X)-Typ b aus Formelschema 1 behandelt.
Ganz besonders vorteilhaft bewirkt dies die Ausrichtung der Aminocellulosen-Polymerketten in mehr oder weniger vertikal angeordneten Polymerkettenbündeln.
Auch diese Ausrichtung ist selbstjustierend, so dass sich die Aminocellulosen-Polymerketten einerseits auf der Substratoberfläche repulsiv ausrichten und andererseits kettenadhäsiv aggregieren. Bei der AFM-Vermessung stellt sich dies auf der Substratoberfläche als typische Polymerkettenbündel bzw. bürstenförmige Topografie dar.
Bei der AFM-Vermessung der modifizierten Substratoberflächen werden zwischen den Polymerketten-Bündeln Mulden- oder Grabentopografien sichtbar.

Besonders vorteilhaft sind diese speziellen topografischen Oberflächenstrukturen für die Biochip-Entwicklung, wenn Substratoberflächen mit einem hohen Grad der Bio-Funktionalisierung benötigt werden.
Denn bei dieser Topografie der Aminocellulosen-Strukturen stehen entlang der Polymerketten mittels NH-(X)-NH₂ an C6 der AGU bei idealisierter Kettenlänge von ca. 100 nm zahlreiche NH₂-Ankergruppen für die Bio-Funktionalisierung zur Verfügung.

Des Weiteren ist vorteilhaft, dass sich Derivat-typische Milieu-Bedingungen zwischen den Polymerketten auf der Substratoberfläche einstellen. Das eröffnet die Möglichkeit, bei der Modifizierung von Substratoberflächen solche Aminocellulosenderivate einzusetzen, die anhand ihrer Strukturen (X) und S sowie der Substitutionsgrade DS_{NH(X)NH2} und DSₛ jeweils biospezifische Milieubedingungen erwarten lassen.

Eine Beeinflussung der Milieubedingungen auf der Substratverbundmaterialoberfläche geht im Falle der kovalenten Bio-Funktionalisierung auch von den NH₂-reaktiven Kopplungsreagenzien aus, wie entsprechende Untersuchungen unter Einsatz von Enzymproteinen als Bio-Funktionsmoleküle zeigten.

### 2.3 Oberflächen-Modifizierung von Substraten mittels hydrophiles SiOₓ-Polymer

Ein Silicoatingverfahren ist überraschenderweise hervorragend geeignet, um Substratmaterialien, die kurzzeitig hitzestabil sind, durch Bildung einer ultradünnen SiOₓ-Polymerstruktur zu hydrophilisieren. Das Kernstück des Verfahrens ist ein Brenner, der mit einem Pyrosilgasgemisch gefüllt ist. Das Gasgemisch enthält eine Silizium-organische Verbindung, die sich bei dem Verfahren unter Bildung eines SiO₂ /Silikat-Gemisches flammenpyrolytisch zersetzt.

Beispielsweise wird auf Glastyp-, Silizium- oder Metall-Substratoberflächen durch kurzzeitiges (< 1 sec) Befächeln mit der Flamme des oben genannten Brenners ein SiO₂ /Silikat-Gemisch gebildet, woraus nach kurzer Zeit ein glasartiges SiOₓ-Polymer als ultradünne und transparente Oberflächenstruktur mit einer Dickenabmessung kleiner 1 - 2 Nanometer entsteht. Die SiOₓ-Polymer-modifizierten Substratoberflächen haben ähnlich geringe RMS-Rauhigkeiten wie die thermisch bei 600°C erzeugten SiO₂-Polymerschichte auf Si-Substratproben.

Das stark hydrophile SiOₓ-Polymer (Wasser-Kontaktwinkel kleiner 10 bis 15 Grad) wird wie erwähnt vorab verwendet, wenn die erfindungsgemäße Bildung des Substratverbundmaterials durch Oberflächen-Modifizierung via Aminocellulosederivaten und/oder NH₂-(Organo)polysiloxanen nicht von vornherein zum Ziel führt.
Die offensichtlich hohe OH-Gruppendichte bzw. Hydrophilie des SiOₓ-Polymers ist aber auch direkt, das heißt ohne weitere Funktionalisierung, für adhäsive Bio-Funktionalisierungen z. B. durch funktionelle Proteine, Zellen oder andere Bio-funktionsmoleküle oder für kovalente Bio-Funktionalisierungen mittels üblicher OH-reaktiver Reagenzien anwendbar.

### 2.4 Variationsmöglichkeiten des Verfahrens

Die Oberflächen-Modifizierung geschieht auf einfache Weise in einem Gemisch der Substratprobe und einer Modifizierungslösung, bestehend aus einem Derivat der Aminocellulosen gemäß Formelschema 1 in bidestilliertem Wasser bzw. Dimethylacetamid (DMA) und/oder aus einem NH₂-(Organo)polysiloxanderivat gemäß Formelschema 2 z. B. in 2-Propanol.

Beispielsweise werden 0,03 bis 0,1%ige filtrierte Derivatlösungen mit den Substratproben in Kontakt gebracht.

Welches Verfahren angewendet wird, richtet sich im Wesentlichen nach dem Substratmaterial und nach der erforderlichen Qualität und dem Anwendungszweck der modifizierten Substrate. Aber auch die Art der Vorbehandlung der Substratoberfläche, z. B. die Reinigung oder eine vorangehende Plasmabehandlung oder eine Modifizierung mittels SiOₓ-Polymer des Substrats spielen hierfür eine Rolle.

Es hat sich bewährt:
- Schütteln des Ansatzes, z.B. 1 Min. bis 6 h,
- Schütteln des Ansatzes im Ultraschallbad, z. B. 5 bis 30 Min.,
- Stehen lassen des Ansatzes, vorzugsweise bei Raumtemperatur,
- Aufschleudern (spin-coating) der Derivat-Lösung,
- Auftropfen der Derivat-Lösung,
- kurzes Eintauchen (dip-coating) in die Derivat-Lösung, oder
- Versprühen (air-brush-Verfahren) der Derivat-Lösung.

Anschließend wird gegebenenfalls mehrmalig mit bidestilliertem Wasser oder DMA oder mit Methanol, Ethanol oder 2-Propanol gewaschen, z.B. unter Schütteln oder im Ultraschallbad. Abschließend wird das Substratverbundmaterial im Argon-Strom getrocknet. Das Substratverbundmaterial bleibt dabei miteinander fixiert. Die Modifizierung der Substratoberflächen ist je nach Substratmaterial und seiner Vorbehandlung in der Regel nach 1 bis 5 Minuten Schütteln, anschließendem intensiven Waschen mit bidestilliertem Wasser bzw. DMA und nachfolgendem Trocknen im Argon-Strom beendet.

Von besonderer Bedeutung für die Nanotechnologie ist die Anwendung des erfindungsgemäßen Verfahrens
- in Kombination mit *µ*CP, zur Herstellung von Substratverbundmaterialien mit nanoskaligen Mustern von Oberflächenstrukturen oder
- bei der Herstellung analytsensitiver Biochips im mikrofluidischen Sensorsystem.

Aus ellipsometrisch ermittelten und mittels AFM verifizierten Dickenabmessungen D≈ 0,5 bis 1 Nanometer von PDA-Cellulosetosylatpolymerketten (s. Formelschema 1) auf Au (111)-Substratoberflächen ist beim Vergleich mit einem kalkulierten Wert D≈0,8 Nanometer für die Dickenabmessung von PDA-Celluloseketten nach Computer-Molekülmodellverfahren zu schließen, dass quasi Monolagen der Aminocellulosenpolymerketten auf die Substratoberfläche übertragen werden.

Mittels computergestützter Kalkulationen des Molekülmodells von Polymerketten-Monolagen für EDA-Cellulose (s. Typ a, i=2, Formelschema 1) ergibt sich D≈ 1 Nanometer und für TEPA-Cellulose (vgl. Typ b, Formelschema 1) D≈3 Nanometer.

Vorteilhaft weisen die modifizierten Substratverbundmaterialien bzw. ihre Oberflächen eine definierte Topografie mit geringer RMS-Rauhigkeit von meist 0,1 bis 1,5 Nanometer auf.

Auch nach kurzzeitiger Behandlung von Substrat mit Modifizierungslösung sind die Oberflächenstrukturen via Derivate gemäß Formelschema 1 und 2 auf der Substratoberfläche fest fixiert, wie Folgereaktionen mit NH₂-reaktiven Kopplungs-Reagenzien, Behandlungen der modifizierten Substratproben mit Lösemitteln bzw. wässrigen Lösungen wechselnder Elektrolyt-Zusammensetzung im Ultraschallbad sowie unter kinetischen Messbedingungen bei massesensitiver Vermessung der modifizierten Substratproben im mikrofluidischen Sensorsystem zeigen.

In einigen Fällen werden die Substratverbundmaterialien z. B. mit einer 1%igen wässrigen Glutaraldehyd (GDA)-Lösung kurzzeitig bei einer Zeitdauer von etwa 10 bis 30 Sekunden behandelt und anschließend mit bidestilliertem Wasser mehrfach gewaschen, frei von überschüssigem GDA.

Der Effekt der partiellen Vernetzung der Oberflächenstruktur bzw. der Aminocellulosenpolymerketten wird auch mittels NH₂-reaktiver Reagenzien anders als GDA erreicht. Die partielle Vernetzung wird beispielsweise im speziellen Falle angewendet, wenn Oberflächenstrukturen mit einer Dickenabmessung größer als 3 Nanometer, das heißt molekulare Multischichten auf der Substratoberfläche erforderlich sind.

Als wesentliche Parameter zur Charakterisierung der Substratverbundmaterialien werden die Dickenabmessungen der Oberflächenstrukturen, die NH₂-Gruppendichte bzw. Konzentration je Substratprobenfläche, der Wasser-Kontaktwinkel und der Wert der RMS-Rauhigkeit herangezogen.

Die NH₂-Gruppen der Oberflächenstrukturen, die mittels Aminocellulosepolymerketten (s. (X), Typ a bis d, Formelschema 1) und/oder NH₂-(Organo)polysiloxan-Polymerketten (s. (X) = a bis c, Formelschema 2) auf die Substratoberflächen lateral übertragen werden, dienen unter anderem als (NH₂-)Ankergruppen für die kovalente Bio-Funktionalisierung. Die Dichte bzw. Konzentration der NH₂-Ankergruppen je Substratprobenfläche ist eine wichtige Maßzahl für die Einsatzmöglichkeit der erfindungsgemäßen Substratverbundmaterialien. Der Wasser-Kontaktwinkel KW dient als Maß für die Hydrophobie- bzw. Hydrophilie-Balance der Oberflächenstrukturen.

In einer weiteren Ausgestaltung der Erfindung werden die Substratoberflächen mit Sauerstoff- bzw. Argon-Plasma für etwa 30 Sekunden bis 2 Minuten vorbehandelt.
Bei nachfolgender erfindungsgemäßer Behandlung mit Modifizierungslösung weisen die erhaltenen Substratverbundmaterialien häufig die jeweils geringsten Dickenabmessungen und RMS-Rauhigkeiten auf.

Vorteilhaft können die modifizierten Substratoberflächen nach ihrer Herstellung zeitlich unbegrenzt aufbewahrt werden
- zur komplettierenden Oberflächenmodifizierung z. B. durch *µ*CP oder durch NH₂-reaktive Reagenzien bzw. Funktionalisierungen zum Zwecke der Einstellung anwendungsspezifischer Oberflächeneigenschaften, wie z.B. pH-Wert, Ladungsverteilung, Hydrophilie- bzw. Hydrophobiebalance, redoxaktiver oder licht-absorbierender Eigenschaften und/oder
- zur elektrostatischen oder kovalenten Kopplung mit Funktionsmolekülen, z. B. von funktionellen Biomolekülen bzw. Proteinen oder Zellen bzw. Zell-Bestandteilen.

Zusammenfassend wird mit der Erfindung erstmals ein einfaches Verfahren für ein umfassendes Strukturdesign von zukunftstechnologisch relevanten Substratmaterialien zur Verfügung gestellt. Dabei stützt sich das Strukturdesign auf die Anwendung
- und Modifizierung der Aminocellulosen-Leitstruktur P-CH2-NH-(X)-NH2 (P-CH2-=Cellulose-C6, s. Formelschema 1) mit den strukturbildenden und biokompatiblen Eigenschaften der Cellulosestruktur, die durch Spacerstrukturen (X) an den AGU-Positionen C6 und Ester-Gruppen S an C2/C3 sowie durch Variation der Substitutionsgrade DSNH(X)NH2 und DSS entlang der Cellulose-Polymerketten leicht und vielfältig modifizierbar sind;
- von speziellen NH2-(Organo) polysiloxanen (vgl. Formelschema 2), insbesondere in Kombination mit µCP;
- vielfältiger NH2-reaktiver bifunktioneller Reagenzien zur weiteren Funktionalisierung, insbesondere Biofunktionalisierung von Substratoberflächen für die biophysikalische und biomedizinische Anwendung.

Durch die Einbeziehung weiterer geeigneter NH₂-Polymere, z. B. Aminopolysaccharide anders als Aminocellulosen nach Formel I, ist das erfindungsgemäße Verfahren noch zu komplettieren.

Im Folgenden werden nur beispielhaft, ohne eine Einschränkung vorzunehmen, Anwendungen des erfindungsgemäßen Verfahrens und der auf diese Weise hergestellten Substratverbundmaterialien beschrieben.

### 3. Anwendungsgebiete

### 3.1 Biophysikalisches Anwendungsgebiet

### 3.1.1 Oberflächen-Modifizierung von Si-Spitzen für Rastersonden-Methoden

Modifizierte Si-Spitzen werden beispielsweise bei der AFM (atomic force microscopy) für die Kraftabstands- bzw. Kraftmodulations-Vermessung von funktionalisierten Substratoberflächen, z. B. Biochipoberflächen, oder bei Rastersondenlithografietechniken zur lateralen Nanostrukturierung von Substratoberflächen, z. B. Biochips, benötigt.

Zur Modifizierung von Si-Spitzen wird folgendermaßen verfahren:
Die Si-Spitze, die an einem so genannten Federbalken (cantilever) befestigt ist, wird anhand dieses cantilevers auf einem Gel-Pack adhäsiv fixiert, so dass die Si-Spitze in den Raum zeigt. Die Si-Spitze wird dann auf eine geeignete Weise vorsichtig gereinigt bzw. vorbehandelt und anschließend mit einer Modifizierungslösung eines geeigneten Derivates gemäß Formelschema 1 oder 2 benetzt. Dabei werden die verfahrensgemäß üblichen Lösungen und Konzentrationen angewendet. Nach einer Benetzungs-Zeitdauer von ca. 30 Min. bis 3 h wird die Modifizierungslösung abgezogen und die modifizierte Si-Spitze mit 30 bis 50 µl Lösungsmittel (z. B. bidestilliertes Wasser, DMA oder 2-Propanol) 3 bis 5 mal gewaschen. Danach wird der am Gel-Pack fixierte cantilever im Argon-Strom getrocknet und anschließend bei AFM-Vermessungen eingesetzt, um den Modifizierungs-Effekt anhand einer geeigneten Probenoberflächen zu charakterisieren.

Der Modifizierungs-Effekt wird besonders deutlich sichtbar, wenn beispielsweise im rasterkraftmikroskopischen Nicht-Kontakt-Modus die modifizierte Si-Spitze mit einer nichtmodifizierten Si-Spitze durch AFM-Vermessung einer Kalibrierprobe, auf deren Oberfläche sich Kegelformen auf Si-Basis mit Abmessungen von wenigen Nanometern befinden, hinsichtlich der abgebildeten topografischen Charakteristika verglichen werden.

Der besondere Effekt bei der Vermessung der Kalibrierprobe ist, dass die jeweils im AFM-Gerät mittels cantilever befindliche Si-Spitze topografisch abgebildet wird. Demnach wird die nicht-modifizierte Si-Spitze in einer typischen kegelförmigen Topografie abgebildet. Das ist bei der beschriebenen modifizierten Si-Spitze nicht der Fall. Hier zeigt die topografische (3D-)Abbildung eine Aufspaltung der Kegelspitze, das heißt eine quasi-Doppelspitze.

Diese Topografie spiegelt die auf der Si-Spitze befindlichen Aminocellulosen-Polymerketten wider. Denn die Vorstellung auf der mikroskopischen Betrachtungsebene ist, dass die adhäsiv fixierten Polymerketten mit einer idealisierten Kettenlänge von ca. 100 Nanometer die Si-Spitze gewissermaßen rundherum fransenförmig überragen, was beim Abrastern der Kalibrierprobe als verlängerte artifizielle bzw. gespaltene Spitze topografisch abgebildet wird.

Besonders vorteilhaft ist die weitere Funktionalisierung der erfindungsgemäß modifizierten Si-Spitzen mittels der genannten NH₂-reaktiven Kopplung mit Funktionsmolekülen, die für den jeweiligen Anwendungsfall der Rastersondentechnik relevant sind. Rastersondenrelevant sind beispielsweise solche Funktionsmoleküle, die Kraft-Abstands- bzw. Kraftmodulations-Effekte bei der AFM-Vermessung von modifizierten Substratoberflächen induzieren oder die bei Rastersondenlithografie-Techniken eine laterale Strukturübertragung via Funktionsmoleküle auf Substratoberflächen ermöglichen.

### 3.1.2 Oberflächenmodifizierung von PDMS-Substraten

Polydimethysiloxane (PDMS) spielen als weiches und leicht verformbares Polymer und aufgrund vorteilhafter physikalischer, chemischer und biokompatibler Eigenschaften in der Mikro- und Nanotechnik, z. B. als Stempel für das µCP, als Biochip-Material speziell in mikrofluidischen Sensorsystemen oder als Implantatmaterial eine besondere Rolle, insbesondere auch wegen der Silan-chemischen Modifizierbarkeit des Sauerstoffplasma-behandelten PDMS. Dabei kommt es darauf an, die PDMS-Oberfläche möglichst anwendungsspezifisch, z. B. analytsensitiv oder biofunktional, zu modifizieren. Durch Behandlung der PDMS-Oberflächen mit Sauerstoff-Plasma entstehen OH-Gruppen (Wasser-Kontaktwinkel kleiner 20 Grad). Die OH-Gruppen werden bekanntermaßen mittels Silan-Verbindungen modifiziert.

Durch das erfindungsgemäße Verfahren werden beispielsweise PDMS-Stempel für das molekulare Bedrucken auf die genannte einfache Weise mit Wechselwirkungs-spezifischen Strukturarealen, wie hydrophobe, elektrostatisch-orientierte oder komplementäre bzw. bioaktive Strukturareale modifiziert. Für alle genannten Anwendungen steht die Derivate-Vielfalt gemäß Formelschema 1 und 2 zur Verfügung. Beispielsweise werden die mit Sauerstoff-Plasma behandelten PDMS-Oberflächen auf die erfindungsgemäß einfache Weise durch kurzen Kontakt mit der jeweiligen Modifizierungslösung modifiziert. Dabei entsteht ein PDMS-Verbundmaterial mit Oberflächenstrukturen kleiner 1-2 Nanometer, RMS-Rauhigkeiten kleiner 0,2-0,5 Nanometer, Wasser-Kontaktwinkeln kleiner 50 bis größer 90 Grad und NH₂-Gruppendichten von 0,5-1,5 nMol je cm² der Substratoberfläche.

### 3.1.3 Erfindungsgemäßes Verfahren mittels µCP

Anhand regelmäßig mikro- oder nanostrukturierter Stempel aus polymeren Materialien werden Oberflächenstrukturen mittels der Derivate gemäß Formelschema 1 und 2 in entsprechenden mikro- bzw. nanoskaligen Mustern auf die Substratoberflächen gestempelt. Geeignete Mikro- und Nanostempel werden vorzugsweise aus Poly(dimethylsiloxan) (PDMS) auf bekannte Weise hergestellt, beispielsweise aus Sylgard 184-Kit, bestehend aus Sylgard-184 A und Sylgard-184 B. Als Stempelmuster dienen beispielsweise parallel angeordnete Linien mit Linienbreiten von 200 nm bis 4 *µ*m und Linienabständen von 200 nm bis 200 *µ*m. Es können auch solche Mikro- bzw. Nanokontaktstempel aus Materialien anders als PDMS angewendet werden.

Im Falle von beispielhaft eingesetzten Si/SiO₂-Substratoberflächen wird beim *µ*CP ausgehend von Aminocellulosederivaten und von NH₂-(Organo)polysiloxanderivaten gemäß Formelschema 1 bzw. 2 folgendermaßen verfahren: Das Linienmuster eines PDMS-Stempels (z. B. Linienabstände: 200 nm bis 50 *µ*m) wird mit 5 bis 10 *µ*l einer 0,05 bis 0,5%igen Lösung eines Derivates der Aminocellulosen-Leitstruktur, z. B. einer PDA-Cellulosetosylat-Lösung in DMA, betropft. Anschließend wird der Stempel 1 bis 5 sec mit der benetzten Seite vorsichtig auf ein Filterpapier gedrückt und danach sofort, vorzugsweise für 2 bis 15 Min., mit einer Si/SiO₂-Substratoberfläche durch leichtes Aufdrücken in Kontakt gebracht. Danach wird der Stempel von der Substratoberfläche entfernt und diese 5 bis 30 Minuten in DMA unter mehrfachem Wechsel der DMA-Phase geschüttelt. Danach wird die gestempelte Substratoberfläche im Argon-Strom getrocknet und das Linienmuster beispielsweise durch (Auflicht-) Mikroskopie (mit Polarisationsfilter), abbildende Ellipsometrie und AFM charakterisiert. Bei der mikroskopischen Betrachtung der gestempelten Substratoberflächen wird das mikroskalige Linienmuster der Oberflächenstrukturen sichtbar und durch abbildende Ellipsometrie werden Linienförmige Oberflächenstrukturen veranschaulicht und vermessen. Es werden Substratverbundmaterialien mit Oberflächenstrukturen < 1 - 3 nm (je nach eingesetztem Derivat) gefunden. Diese ellipsometrisch bestimmten Dickenabmessungen der Oberflächenstrukturen werden durch AFM betätigt. Mittels AFM werden Linienbreiten gefunden, die mit den Sollwerten von 200 nm bis 4 *µ*m der eingesetzten Mikro- bzw. Nanostempel in Einklang stehen.

Das *µ*CP-Verfahren wird bei den oben definierten Substratoberflächen auch mit Modifizierungslösungen von NH₂(Organo)polysiloxanderivaten P1 bis P5 gemäß Formelschema 2 angewendet.

Beispielsweise wird auf Si/SiO₂-Substratoberflächen durch *µ*CP ein Linien-Strukturmuster in Abständen von 2 bis 200 *µ*m gestempelt. Dazu wird der PDMS-Stempel beispielsweise mit 5 bis 10 *µ*l einer 0,03 bis 0,1%igen Lösung eines NH₂-(Organo) polysiloxanes (z.B. Typ P2b) in 2-Propanol betropft und danach wie beim *µ*CP mit Aminocellulosederivat verfahren. Nach Entfernen des Stempels von der Substratoberfläche wird diese 5 bis 15 Minuten in 2-Propanol unter mehrfachem Wechsel der 2-Propanol-Phase geschüttelt. Danach wird die gestempelte Substratprobe im Argon-Strom getrocknet und das Linienmuster auf die gleiche Weise wie beim *µ*CP mittels Aminocellulosederivat charakterisiert. Mittels AFM werden linienförmige Oberflächenstrukturen mit Dickenabmessungen < 1 - 2 nm und Linienbreiten gefunden, die den Sollwerten des Stempels entsprechen.

Beide Verfahren, also das erfindungsgemäße Verfahren und Varianten des *µ*CP, ermöglichen auf synergetische Weise Substratoberflächen mit Eigenschafts- bzw. Wechselwirkungs-Mustern beispielsweise für Bio-Funktionalisierungen bzw. biophysikalische Anwendungen oder für Protein- und Zell-Adhäsion und so weiter nach den Kriterien eines einzelnen Anwendungsfalles zu optimieren.

Dabei werden die Derivate gemäß Formelschema 1 und 2 eingesetzt. Mittels entsprechender Mikro- oder Nano-Stempel lassen sich auch Strukturmuster anders als Linienmuster auf den Substratoberflächen herstellen. Die Varianten-Vielfalt, die das erfindungsgemäße Verfahren und das *µ*CP eröffnen, wird im Folgenden anhand von Beispielen dargelegt:

Beispiel 1: Eine Substratoberfläche wird je nach Anwendung mittels eines Aminocellulosederivates der Cu-Chelat-bildenden Typ-Reihe b (z.B. TETAT-Cellulosederivat) erfindungsgemäß modifiziert und anschließend durch *µ*CP ein Muster aus PDA-Cellulosetosylat (mit redoxchromogenen Eigenschaften) gestempelt. An die NH₂-Ankergruppen des PDA-Restes an C6 (der AGU) wird beispielsweise ein redoxaktives Protein, wie ein sog. Cu-Protein, immobilisiert.

Beispiel 2: Eine Substratoberfläche wird je nach Anwendungserfordernis entweder durch ein Aminocellulosederivat aus der Typ-Reihe b, z. B. ein DPTA-Cellulosederivat zwecks Bildung von Cu-Chelaten oder Einstellung eines Protein relevanten pH-Wertes oder mittels eines redoxaktiven Aminocellulosederivates der Typ-Reihe c oder d erfindungsgemäß modifiziert und anschließend mittels *µ*CP ein Muster aus einem Aminocellulosederivat des Typs a mit Spacereffekt oder aus einem NH₂(Organo)-polysiloxanderivat P1 bis P5 gestempelt. Die freien NH₂-Ankergruppen werden durch ein NH₂-reaktives Kopplungsreagenz, das auf die Bio-Funktion abgestimmt ist, bio-funktionalisiert.

Beispiel 3: Eine Substratoberfläche wird je nach Anwendungserfordernis mittels SiOₓ-Polymer hydrophilisiert und anschließend mittels *µ*CP ein Muster aus einem Aminocellulosederivat der Typ-Reihen a oder b bzw. einem NH₂-(Organo)polysiloxanderivat P1 bis P5 gestempelt, um an die freien NH₂-Ankergruppen eine strukturell abgestimmte Biomolekülspezies via üblicher NH₂-reaktiver Kopplungsreagenzien zu immobilisieren.

Beispiel 4: Eine Substratoberfläche wird je nach Anwendungserfordernis mittels eines NH₂-(Organo)polysiloxanderivates P1 bis P5 erfindungsgemäß modifiziert und anschließend mittels *µ*CP ein Muster aus einem Aminocellulosederivat oder einem Derivat-Gemisch der Typ-Reihen a bis c gestempelt, um durch NH₂-reaktiver Folgereaktion biorelevante Eigenschaften (z. B. pH-Wert, Ladungsverteilung, Wasser-Kontaktwinkel) einzustellen und/oder (Bio-)Funktionsmoleküle zu immobilisieren.

Bei einer anderen *µ*CP-Ausführungsvariante wird die Stempeloberfläche mit der Derivat-Lösung (als "Stempel-Tinte") mittels Stempelkissen benetzt. Beispielsweise wird ein PDMS-Stempelkissen (Abmessung ca. 10x10 mm, Dicke ca. 1 bis 3 mm) aus dem "PDMS Sylgard 184"-Material gegossen, danach mit der Modifizierungslösung ca. 3 h unter Rühren getränkt und anschließend 1 bis 2 Min. im Argon-Strom getrocknet. Der PDMS-Stempel wird auf das vorbehandelte Stempelkissen gedrückt, und anschließend mit der Substratoberfläche ca. 2 Minuten in Kontakt gebracht. Die gestempelte Substratprobe wird dann, wie oben beschrieben, behandelt und charakterisiert.

### 3.1.4 Oberflächenstrukturmuster mit Gold-Nanopartikeln

An Strukturmuster, die mittels *µ*CP aus Derivaten gemäß Formelschema 1 oder 2 auf einer Substratoberfläche gestempelt wurden, lassen sich Gold-Nanopartikel bei Kontakt mit kommerziell erhältlichen Gold-Kolloid-Lösungen adhäsiv fixieren. Auf einer Substratoberfläche werden beispielsweise alternierende Strukturmuster einerseits zur Adhäsion von Gold-Nanopartikeln und andererseits für die Bio-Funktionalisierung hergestellt. Hierbei werden solche Derivate gemäß Formelschema 1 und 2 angewendet, die den strukturellen bzw. funktionellen Erfordernissen der eingesetzten Bio-Funktion entsprechen. Gold-Nanopartikel spielen, z. B. auf Substratoberflächen, in Verbindung mit funktionellen Biomolekülen bzw. Proteinen bei der Biochip-Entwicklung bzw. bei bioelektronischen Funktionsbausteinen eine wichtige Rolle.

Bei der erfindungsgemäßen Herstellung einer Substratoberfläche, die ein Strukturmuster aus adhäsiv fixierten Gold-Nanopartikeln und eine Bio-Funktion enthält wird beispielsweise folgendermaßen verfahren: Eine Substratoberfläche wird mit einem Derivat gemäß Formelschema 1 oder 2 bestempelt. Danach wird die gestempelte Substratoberfläche mit einer kommerziell erhältlichen Gold-Kolloid-Lösung (Gold-Nanopartikel: 3 bis 30 nm) behandelt und anschließend mit einer Biomolekülspezifischen Oberflächenstruktur aus einem Derivat gemäß Formelschema 1 oder 2 erfindungsgemäß modifiziert. Danach werden an die NH₂-Ankergruppen der modifizierten Substratoberfläche via NH₂-reaktiver bifunktioneller Reagenzien Bio-Funktionsmoleküle kovalent gekoppelt.

Alternativ wird eine Substratoberfläche je nach biospezifischem Erfordernis z. B. mittels SiOₓ-Polymer hydrophilisiert und anschließend mittels eines Kupfer(Cu)-Chelat-bildenden Aminocellulosederivates des Typs b gemäß Formelschema 1 modifiziert und anschließend mit einer Cu-Ionen-Lösung behandelt. Danach wird die Cu-Ionen-modifizierte Substratoberfläche beispielsweise durch *µ*CP mit einer Oberflächenstruktur aus einem Derivat gemäß Formelschema 1 oder 2, z. B. in Linienform, gestempelt und anschließend mit einer kommerziell erhältlichen Gold-Kolloid-Lösung (Gold-Nanopartikel:3 bis 30 nm) behandelt. Die so modifizierte Substratoberfläche wird entweder via eines bio-spezifischen NH₂-reaktiven Kopplungsreagenz bio-funktionalisiert oder die Substratprobe wird nochmals mit einer Modifizierungslösung eines Derivats gemäß Formelschema 1 oder 2 zwecks adhäsiver Kopplung an die Gold-Nanopartikel behandelt und danach auf die übliche Weise ein Bio-Funktionsmolekül, z. B. DNA-Sequenzen, kovalent immobilisiert.

### 3.1.5 Biochips

SAW-Chips bestehen aus Quarzscheiben, die aus einem (Quarz-) Einkristall geschnitten werden. Auf der Quarzoberfläche befindet sich eine SiO₂-Polymerschicht mit einer Dickenabmessung von ca. 5 *µ*m als Signal-führende Schicht. Mittels Silanchemischer Verfahren gemäß Stand der Technik, ist es nicht möglich, eine reproduzierbare Kopplung von Bio-Funktionsmolekülen, z. B. DNA- oder RNA-Aptameren, auf der SiO₂-Polymeroberfläche der SAW-Chips herzustellen.

Besonders vorteilhaft ermöglicht nun das erfindungsgemäße Verfahren die Oberflächen-Modifizierung des SAW-Chips bis hin zu einem funktionsfähigen Biochip direkt auf der Signalführenden SiO₂-Polymerschicht oder auf auch einer darauf befindlichen Gold (Au)-Schicht auch dann, wenn sich der SAW-Chip im mikrofluidischen Sensorsystem befindet. Im Falle eines Au-beschichteten SAW-Chips wird die Au-Oberfläche vor Anwendung des erfindungsgemäßen Verfahrens auf die übliche Weise gereinigt bzw. vorbehandelt, z. B. mittels Argon-Plasma.

Analyt-sensitive SAW-Chips werden beispielsweise auf der Signal-führenden SiO₂-Polymerschicht in folgenden Schritten im mikrofluidischen Sensorsystem hergestellt:
Schritt 1: Beispielweise wird eine 0,5%ige wässrige TETAT-Cellulosetosylat-Lösung über den SAW-Chip geleitet (Fließgeschwindigkeit ca. 25 *µ*l/Min., Fließdauer von ca. 9 Min.). Die als übliche Meßgröße beobachtete Phasenänderung, das heißt die Masse-Zunahme, des SAW-Chips ist nach ca. 3 Min. beendet. Danach wird bidest. Wasser durch das mikrofluidische Sensorsystem geleitet (Fließgeschwindigkeit ca. 25 *µ*l/Min., Fließdauer ca. 9 Min.) - zwecks Ablösung von ggf. nicht-adhäsiv auf der Chipoberfläche befindlichem TETAT-Cellulose-tosylat. Dabei wird kaum eine Signaländerung, d. h. kaum Masse-Ablösung, beobachtet. Der Schritt 1 wird mit gleicher TETAT-Cellulosetosylat-Lösung bei gleichen Fließbedingungen wiederholt. Es wird keine Masse- bzw. Signaländerung des SAW-Chips beobachtet - auch nicht beim anschließenden Durchleiten von bidest. Wasser (Fließbedingungen wie oben). D. h. die Modifizierung der SAW-Chipoberfläche ist mittels TETAT-Cellulose-tosylat-Lösung innerhalb einer Fließdauer von 3 Min. beendet.
Schritt 2: Die Aminocellulosen-modifizierte SAW-Chipoberfläche wird mittels einer üblichen NH₂-reaktiven bifunktionellen Reagenz, z. B. Glutaraldehyd (GDA), funktionalisiert. Dazu wird eine 25%ige wässrige GDA-Lösung über die modifizierte SAW-Chipoberfläche geleitet (Fließgeschwindigkeit ca. 50 *µ*l/min., Fließdauer ca. 5 Min.). Anschließend wird die nicht an der SAW-Chipoberfläche umgesetzte bifunktionelle Reagenz mittels bidest. Wasser bei gleicher Fließgeschwindigkeit und Dauer entfernt. Die vermessene Phasenänderung bzw. Masse-Zunahme signalisiert, dass die SAW-Chipoberfläche via GDA funktionalisiert wurde.
Schritt 3: Ausgehend von der GDA-funktionalisierten SAW-Chipoberfläche wird mittels eines Anti-Thrombin-RNA-Aptamers ein Analyt (Thrombin)-sensitiver SAW-Chip (SAW-Sensorchip) hergestellt. Dazu wird eine Anti-Thrombin-RNA-Aptamer-Lösung in bidest. Wasser (1 *µ*molar) über die SAW-Chipoberfläche geleitet (Fließgeschwindigkeit ca. 25 *µ*l/Min., Fließdauer ca. 9 Min.). Die resultierende Phasenänderung signalisiert, dass das Aptamer auf der SAW-Chipoberfläche fixiert vorliegt. Bei anschließendem Durchleiten von bidest. Wasser (Fließgeschwindigkeit ca. 25 *µ*l/Min., Fließdauer ca. 9 Min.) zeigt sich, dass das Aptamer nicht abgelöst wird. Der SAW-Sensorchip ist einsatzbereit, um Thrombin als Analyt zu messen.
Sensor-Test- bzw. Messschritt: Der Test- bzw. Messstatus des mikrofluidischen Sensorsystems wird durch einen SELEX-Puffer (1 mmolar, pH 8) mit einer Fließgeschwindigkeit von ca. 25 *µ*l/Min. eingestellt. Der SAW-Sensorchip ist Thrombinspezifisch und frei von unspezifischer Protein-Bindung, wie sich anhand von Testläufen mittels Thrombin- bzw. Elastase- und Rinderserumalbumin-Lösungen in SELEX-Puffer erweist. Das Thrombin, welches sich nach dem Messschritt auf der Sensoroberfläche befindet, wird mittels 0,1 molarer NaOH-Lösung abgelöst. Die anschließende erneute Vermessung der Thrombin-Lösung in SELEX-Puffer bestätigt, dass der SAW-Sensorchip regenerierbar ist und reproduzierbare Messwerte liefert.

Auf die beschriebene Weise lassen sich auch Sensor-Chipoberflächen für Messprinzipien anders als das SAW-Prinzip unter Bedingungen eines mikrofluidischen Sensorsystems durch das erfindungsgemäße Verfahren modifizieren. Dabei können die Sensorchips aus unterschiedlichen Substratmaterialien bestehen, wie unter 2. definiert. Bezüglich der Oberflächenstrukturen steht mindestens die gesamte Derivatevielfalt gemäß Formelschema 1 und 2 zur Verfügung. Darüber hinaus lässt sich die strukturelle Varianz durch zusätzliche Einbeziehung weiterer Diamine, Oligoamine oder Polyamine in die allgemeine Derivatisierungs-Prozedur in einem erheblichen Umfang noch komplettieren, wie bereits an anderer Stelle erwähnt.

### 3.2 Biomedizinisches Anwendungsgebiet

### 3.2.1 Strukturdesign von Substratoberflächen für in vitro Zell-Kulturen bzw. Zell-Adhäsionen

Die Adhäsion oder Abstoßung von Proteinen bzw. von lebenden Zellen auf Grenzflächen bzw. Substratoberflächen sind äußerst komplexe Prozesse. Zwecks Untersuchung von Zusammenhängen über Zell-Adhäsion, Zell-Wachstum, Zell-Differenzierung, programmierter Zelltod anhand von in vitro Zellkulturen auf Oberflächen mit dem Ziel der medizinischen Implantat-Entwicklungen nach dem Prinzip des Tissue Engineering oder der biophysikalischen Anwendung von Zellen (z. B. von Neuronen u.a.), wird nach Einflussfaktoren anhand von modifizierten Substratoberflächen gesucht.
Die strukturelle Heterogenität der bisher bekannten modifizierten Substratoberflächen für in vitro Zellkulturen bzw. Zell-Adhäsionen ist für das Erkennen diesbezüglicher Zusammenhänge wenig geeignet. So werden zur Herstellung von lateralen Kontrasten, z. B. von alternierenden Strukturarealen mit hydrophoben bzw. hydrophilen Eigenschaften auf Substratoberflächen häufig Poly- und Oligo(ethylenglykole), Dextranen und so weiter als Protein- und Zell-resistente Matrixstrukturen eingesetzt.

Um Zell-Adhäsionen zu untersuchen, werden auch häufig Substratoberflächen mit definierten Mustern adhäsionsfördernder Proteine, wie extrazelluläre Proteine, Proteoglykane, Kollagene mit sich wiederholenden Sequenzen Gly-Pro-Pro oder Fibronektin, Fibrinogen, Laminin und so weiter, eingesetzt, welche mit methylterminierten Oberflächenarealen in Wechselwirkung treten. Alternativ kann die Kopplung aber auch über Oligopeptide mit Zell-Adhäsionsarealen oder kovalent sowie unspezifisch gebundenen Antikörpern erfolgen.

Die Ausbildung von kovalenten Bindungen sowie Wechselwirkungen über Dehydratisierung hydrophober Oberflächenareale zwischen Substratoberfläche und Proteinen bzw. Zellen sind wichtige Aspekte für die Funktion von Proteinen und Zellen gegenüber Inaktivität, Verdrängung oder Reorganisation unter lateraler Verschiebung von Oberflächenstrukturen. Die Komplexität wird bei Zell-Kontakten dadurch noch erhöht, weil Proteine und Stoffe der Zelle und des Kulturmediums mit der Substratoberfläche in Wechselwirkung treten. Die Wechselwirkungen sind Wasserstoff-Brücken-getrieben oder von elektrostatischer, van der Waals'scher (dispersiver) sowie kovalenter Natur.

Im Licht einer völlig neuen Entwicklung in der Implantat-Technik, die seit einigen Jahren auf die Nutzung von Zellkulturtechniken abzielt, gewinnen Verfahren für ein Strukturdesign von Zell-spezifischen Substratoberflächen für die Biomaterial-Entwicklung eine enorme Bedeutung. Beim Tissue Engineering werden neue Organe ausgehend von funktionalen Zellen auf Zell-spezifischen Trägerstrukturen außerhalb des Körpers gebildet, um sie einem Patienten einzupflanzen. An das Tissue Engineering sind hohe Erwartungen für die Zukunft der Implantat-Entwicklung geknüpft. Ziel ist die Herstellung von Oberflächen, die die Funktion der extrazellulären Matrix simulieren und mit dem Empfängergewebe auf Rezeptorbasis spezifische Reaktionen eingehen.

Durch das erfindungsgemäße Verfahren ist es in Kombination mit dem *µ*CP erstmals möglich, Substratmaterial-Oberflächen, insbesondere auch solche für Implantationszwecke, auf der Grundlage der Derivatisierung einer natürlichen polymeren Leitstruktur des Aminocellulosen-Typs der allgemeinen Formel I im Sinne von Fragestellungen zur Wechselwirkung zwischen Zellen und Substratoberfläche strukturell zu modellieren.

### 3.2.2 Oberflächenstrukturdesign von Implantatmaterialien

Auch die herkömmliche Implantat-Entwicklung auf der Grundlage von geeigneten Metallen/Metalloxiden und Legierungen, Keramiken oder Polymeren bzw. textilen Materialien erfordert Verfahren der Oberflächen-Modifizierung, um die Biofunktionalität der Implantatoberfläche zu erhöhen und die Vorgänge an den Grenzflächen Implantat/Gewebe oder Implantat/Blut so weit wie möglich zu kontrollieren bzw. das Einwachsverhalten der Implantate zu optimieren. Dabei geht es vor allem darum, zwei wesentliche Gefahren Immunreaktionen und die Blut-Gerinnungskaskade bei Implantaten, z. B. Stents (künstliche Gefäßstützen), artifizielle Gefäße, Stütz-Implantate und so weiter weitgehend zu vermeiden.

Zwei wesentliche Wege werden beschritten:
1. die Entwicklung von nanostrukturierten biofunktionalen Oberflächen mit verbessertem Blut- bzw. Einwachsverhalten und
2. die Einkopplung von biologischen Signalen auf die Implantatoberfläche zur aktiven Steuerung des Zellwachstums. Das heißt, es werden Oberflächenstrukturen benötigt, die nanotechnologisch so gestaltet sind, dass auf ihnen Zellen besonders gut wachsen oder je nach Anwendungszweck sich gerade nicht ansiedeln können, wie z. B. bei Stents, bei denen häufig die Gefahr von Reststenosen besteht. Zur Frage, welche Oberflächenstrukturen optimale biofunktionale bzw. biokompatible Eigenschaften haben, gibt es nach dem Stand der Technik verschiedene Vorstellungen, die auch von den Anwendungsbedingungen und der Verweildauer des Implantates abhängen. Eine Rolle spielen beispielsweise hydrophobe Oberflächen mit Lotus-Effekt oder irreversible Passivierung durch Protein, z. B. Albumin, hydrophile oder negativ geladene Oberflächen (Minimierung der Protein-Adhäsion)oder Oberflächen mit Funktionsmolekülen, z. B. Antithrombotika, wie Heparin, Fondaparinux, Induronsäure und so weiter. Aber auch die Topografie (Rauhigkeit) der Implantatoberfläche hat einen Einfluss auf die Biokompatibilität.

Das erfindungsgemäße Verfahren des Oberflächenstrukturdesigns bietet alle Voraussetzungen, um den genannten Herausforderungen der Implantatoberflächen-Modifizierung zu genügen. Das trifft sowohl auf die Einbeziehung der verschiedenen Implantatmaterialien, als auch auf die Herstellung der strukturellen Vielfalt der biofunktionalen Oberflächen-Eigenschaften zu.

Auf folgende Implantatmaterialien ist das erfindungsgemäße Verfahren prinzipiell anwendbar: Edelstahl, Chrom-, Kobalt-, Nickel-Legierungen, Gold, Platin, Titan/Titanoxid, Tantal/Tantaloxid, Keramiken, Keramik-Zirkonoxid, Cellulosen bzw. Bakteriencellulosen in Flächen- oder Röhren-Form, Poly(dimethylsiloxane) (PDMS), Polymethylmethacrylat (PMMA, Plexiglas),Poly-N-isopropylacrylamid (PNiPAM), Poly-Glykolid-co-Lactid (PGL), Polymere mit Carboxyl- oder Sulfo-Gruppen, wie Polyhydroxiethylmethacrylat (PHEMA).

Vorteilhaft ist das erfindungsgemäße Verfahren geeignet, um auf unterschiedlichen Substratmaterialien, insbesondere auf den genannten Implantatmaterialien, Strukturmuster bzw. Strukturareale herzustellen, denen z. B. hydrophobe bzw. dispersive, hydrophile, elektrostatische bzw. reaktive Eigenschaften sowie Spacereffekte oder geringe Rauhigkeiten, inhärent sind. Darüber hinaus können an die Oberflächen der Implantat-Verbundmaterialien via genannte NH₂-reaktive bifunktionelle Reagenzien Funktionsmoleküle zur Verbesserung der Biokompatibilität gekoppelt werden. Beispielsweise sind die Oberflächenstrukturen aus den Derivaten gemäß Formelschema 1 und 2 von vornherein geeignet um die Carboxyl- bzw. Sulfofunktionalisierten Antithrombotika, wie Heparin, Fondaparinux, Induronsäure, elektrostatisch fest zu fixieren.

Hervorzuheben an dieser Stelle ist das wesentliche Neue, dass die strukturelle Oberflächen-Modellierung von unterschiedlichen Substratmaterialien insbesondere anhand von Derivaten (gemäß Formelschema 1) ein und derselben Polymerstruktur, nämlich z. B. der Cellulosestruktur geschieht.

Vorteilhaft werden bei der Derivatisierung der Leitstruktur gemäß Formel I die funktionellen Eigenschaften an AGU-Positionen C6 - entlang der Polymerketten - modifiziert, aber die gemeinsamen Grundeigenschaften, wie die biokompatiblen, strukturbildenden, konformationellen, adhäsiven Eigenschaften, bleiben erhalten.

Darüber hinaus werden alle Derivate der allgemeinen Formel I als konformationell uniforme Polymerketten unter Erhalt der oben genannten Grundeigenschaften auf die gleiche Weise durch spontane Adhäsion und Selbstorganisation mit der genannten Monolagen-ähnlichen Dickenabmessung auf unterschiedliche Substratmaterial-Oberflächen lateral übertragen. Schließlich werden die vielfältig modifizierbaren Strukturmerkmale bzw. Wechselwirkungsareale der (Aminocellulosen-) Polymerketten durch *µ*CP in mikro- bzw. nanoskaligen Mustern auf die Substratmaterial-Oberflächen lateral übertragen. Die Möglichkeiten der Oberflächen-Modifizierung von Substratmaterialien werden durch die Kombination der Leitstruktur-Derivate mit NH₂-Oligo)polysiloxanen und/oder mit der SiOₓ-Polymer-Modifizierung erheblich erweitert.

Des weiteren ist die Vielfalt der Leitstruktur-Derivate durch die Beispiele gemäß Formelschema 1 bei weitem nicht erschöpft, sondern durch Einbeziehung weiterer Diamine, Oligoamine oder Polyamine in die allgemeine Derivatisierungs-Prozedur lassen sich letztlich die Möglichkeiten der strukturellen Modellierung von Substratmaterial- bzw. Implantatmaterial-Oberflächen noch in einem erheblichen Maße komplettieren.

Aus genannten Ergebnissen sind Aminocellulosen-modifizierte Substratverbundmaterialien bekannt, die frei von unspezifischen Protein-Adhäsionen bleiben. Andererseits ist auch bekannt, dass die erfindungsgemäßen Substratverbundmaterialien für die Bio-Funktionalisierung, z. B. für die Kopplung mit empfindlichen Enzymproteinen, DNA- oder RNA-Apatameren unter Erhalt ihrer biologischen Funktion, hervorragend geeignet sind. Die Ergebnisse und die weiterhin genannten vorteilhaften Oberflächen-Eigenschaften der Substratverbundmaterialien bilden die Grundlage für ein biokompatibles Oberflächenstrukturdesign von verschieden Implantatmaterialien bzw. Biomaterialien.

### 3.2.3 Anwendung des erfindungsgemäßen Verfahrens für Wirkstoff-Carrier

In der Diagnostik bzw. Therapie verbindet man große Hoffnungen mit Drug-Delivery-Systemen. Das sind z. B. Oberflächenmodifizierte Nanopartikel aus SiO₂- oder Metalloxid-Nanopartikeln, die als Vehikel für den Wirkstofftransport an den Wirkort im Körper des Menschen dienen.
Vorteilhaft kann mit dem erfindungsgemäßen Verfahren die erforderliche Wirkstoff-Trägereigenschaft durch Oberflächen-Modifizierung der Nanopartikel bereit gestellt werden.

Im Weiteren wird die Erfindung an Hand spezifischer Ausführungsbeispiele näher beschrieben.

### Substratproben auf Silizium (Si)-Basis

Als Substrate des Si-Typs wurden rechteckige oder runde Si-Substratproben der Abmessung 6x6 mm bzw. D= 10 mm eingesetzt. Es wurden Si-Substratproben mit nativer Siliziumoxid- bzw. SiO₂-Polymer-Schicht und solche mit thermisch oder pyrolytisch erzeugter SiO₂- bzw. SiOₓ- Polymerschicht bei variierender Dicken-Abmessung < 1 - 2 nm oder 6 nm (thermisch) bis 6 *µ*m (im Falle von SAW-Chips) eingesetzt.

Als Glastyp-Substrate wurden rechteckige und runde Mikroskopie-Deckgläschen (MD) mit einer Abmessung von 10x10 mm bzw. D= 10 mm eingesetzt. Die MD können vor dem Einsatz mit einem Reinigungsmittel wie Extran-Lösung, und/oder Aceton im Ultraschallbad bei 10 Minuten vorbehandelt. Auch eine Vorbehandlung mit Piranha-Lösung oder mit konzentrierter Schwefelsäure oder Salpetersäure bei 10 bis 30 Minuten und anschließender Spülung mit bidest. Wasser ist möglich. Die Proben wurden sodann mit Sauerstoff- oder Argon-Plasma für ca. 1 bis 2 Minuten behandelt.

Metalloxid-Substratproben (Al-Oxid)Si-Chips (10x10 mm) wurden mittels Pulsed Laser Deposition (PLD) mit Aluminiumoxid (Al-Oxid) beschichtet (Dickenabmessung= 5 bis 6 nm; KW: 70 bis 80°; RMS-Rauhigkeit:0,1 bis 0,15 nm) und ohne Vorbehandlung eingesetzt.

### Au-Substratproben

Ein Si-Wafer wurde im Schritt 1 mit Chrom, (Dickenabmessung ca. 2-3 nm) und im Schritt 2 auf bekannte Weise mit einer Goldschicht besputtert bzw. bedampft (Dickenabmessung ca. 100 nm). Anschließend wurde der Gold-beschichtete Si-Wafer in rechteckige oder runde Au-Substratproben der Abmessung 6x6 mm bzw. D= 10 mm geschnitten. Die Au-Substratproben wurden vor dem Einsatz auf unterschiedliche Weise vorbehandelt, z. B. mit einer 5%-igen wässrigen Lösung von Extran-Reinigungsmittel bei 1 bis 5 Minuten im Ultraschallbad behandelt. Danach wurde mit bidest. Wasser und abs. Ethanol gewaschen und anschließend im Argon-Strom getrocknet oder mit Piranha-Lösung behandelt und anschließend, wie oben genannt, gewaschen und getrocknet oder mit Sauerstoff- bzw. Argon-Plasma behandelt, um bei der erfindungsgemäßen Oberflächen-Modifizierung eine besonders geringe RMS-Rauhigkeit bzw. hohe Qualität zu erzielen.

### Au(111)-Substratproben

Au(111)-Probenflächen (D= ca.5 mm) wurden poliert. Vor jedem Einsatz wurden die Au(111)-Probenflächen ca. 12 bis 24 h mit konz. Schwefelsäure behandelt, anschließend mit bidest. Wasser und abs. Ethanol gewaschen und nach dem Trocknen im Argon-Strom mittels eines Butangas-Brenners vorsichtig bis zur Gelbglut für eine Dauer von 5 bis 10 Minuten geglüht. Die Au(111)-Probenflächen wurden nach Abkühlung auf Raumtemperatur sofort eingesetzt oder vor dem Einsatz noch mit Piranha-Lösung behandelt.

### PDMS-Substratproben

PDMS-Substratproben wurden mittels des Sylgard 184-Kits (Firma "Dow Corning"), bestehend aus Sylgard-184 A und Sylgard-184 B auf Si-Chips hergestellt. Dazu wurden "A" und "B" im Verhältnis 10:1 gemischt und mit Hexan (1:1000) verdünnt. Aus je 5 *µ*l dieses Gemisches wurden die PDMS-Substratproben mittels spin-coating (20 Tausend U/Min.) auf runden Si-Chips (D= 10 mm, PDMS-Dickenabmessung: 2 bis 4 nm, ellipsometrisch) bereitet. Für den weiteren Einsatz wurden die PDMS-Substratproben mit Sauerstoff-Plasma vorsichtig behandelt (Dauer ca. 30 sec und Abdeckung der Probe mit einem Metallsieb). Nach Behandlung mit Sauerstoff-Plasma betrugen die PDMS-Schichtdicken 1 bis 2 nm. Die PDMS-Substratproben wurden sofort bei der erfindungsgemäßen Oberflächen-Modifizierung eingesetzt.

### Behandlung mit hydrophilem SiOₓ-Polymer

Es wurde ein kommerzielles Silicoating-Verfahren der Klebe- und Zahntechnik angewendet, um kurzzeitig hitzestabile Substratmaterialien durch Bildung eines ultradünnen SiOₓ-Polymer-Filmes zu hydrophilisieren. Dazu wurde die Substratmaterial-Oberfläche mit der Flamme eines feuerzeugähnlichen Gerätes, das ein brennbares "Pyrosil"-Gasgemisch mit einer Silizium-organischen Verbindung enthielt, extrem kurzzeitig (< 1 sec) befächelt. Das dabei gebildete SiO₂/Silikat-Gemisch wurde nach kurzer Zeit ein glasartiges "SiOₓ-Polymer" als ultradünner und transparenter Film (Dickenabmessung < 1 bis 2 nm). Das Verfahren war bei allen kurzzeitig (< 1 sec) stabilen Substratmaterialien anwendbar.

### Oberflächenmodifizierte Substrate (Substratverbundmaterialien)

Allgemeine Verfahrens-Vorschrift: Für Substratverbundmaterialien mit besonders geringer RMS-Rauhigkeit bzw. höchster Oberflächenqualität wurden die Substratoberflächen mittels Sauerstoff-Plasma vorbehandelt.

Die Oberflächen-Modifizierung bzw. das Substratverbundmaterial wurde mittels einer sog Modifizierungslösung hergestellt, mit der die Substratproben auf unterschiedliche Weise und Zeitdauer in Kontakt gebracht werden. Die Modifizierungslösung bestand entweder aus einer 0,05 bis 0,5%igen Lösung eines Aminocellulosederivates gemäß allgemeiner Formel I bzw. Formelschema 1 in bidestilliertem Wasser oder Dimethylacetamid (DMA) oder aus einer 0,03 bis 0,1%igen Lösung eines NH₂-(Organo)polysiloxanderivat P1 bis P5 gemäß Formelschema 2 in Methanol, Ethanol oder 2-Propanol. Die Modifizierungslösungen wurden vor ihrem Einsatz vorzugsweise mittels Zentrifugal-Filter-Röhrchen (Porenweite ca. 0,2 bis 0,45 *µ*m) filtriert. Für Oberflächen-Modifizierungen wurden je nach Anwendungszweck, Substratmaterial und Vorbehandlung unterschiedliche Prozedur-Varianten angewendet, z. B.
- Schütteln (1 Min. bis 6 h)
- Schütteln im Ultraschallbad ( 5 bis 30 Min.)
- Stehen (1 bis 12 h)
- spin-coating (1 bis 20 Tausend Umdrehungen pro Min.)
- Auftropfen und 5 bis 10 Min. Verweildauer
- dip-coating (5 bis 60 sec)
- air-brush und 5 bis 10 Min. Verweildauer
- Mikro- und Nanokontakt-Drucken (*µ*CP).

Im Falle des *µ*CP wurde die Modifizierungslösung mittels eines mikro- oder nanostrukturierten Stempels aus einem Polymer, vorzugsweise aus PDMS, mit der Substratoberfläche in Kontakt gebracht. Die PDMS-Stempel wurden auf übliche Weise aus dem kommerziellen "PDMS-Sylgard 184", z.B. je nach Anwendungszweck mit mikro- oder nanostrukturierten Linienmustern bzw. Linienabständen angefertigt. Die Modifizierungslösung wurde als Stempeltinte auf unterschiedliche Weise auf die Stempeloberfläche aufgebracht.

Die ganzflächig oder in Mustern Oberflächen-modifizierten Substratproben bzw. Substratverbundmaterialien wurden mit dem jeweiligen Lösungsmittel mehrfach und intensiv unter Schütteln, z.B. im Ultraschallbad, frei von nicht adhäsiv auf der Substratoberfläche fixiertem Derivat gewaschen und nachfolgend im Argon-Strom getrocknet.

Die Modifizierungs-Prozedur war je nach Typ und Vorbehandlung der Substratproben und nach Maßgabe der erforderlichen Modifizierungs-Charakteristika, z. B. Dickenabmessung der Oberflächenstruktur, NH₂-Gruppen-Konzentration/Fläche, RMS-Rauhigkeit u.a., meist nach 1 bis 5 Min. Schütteln, anschließendem Waschen mit Lösungsmittel und nachfolgendem Trocknen im Argon-Strom beendet.

Die modifizierten Substratproben wurden unmittelbar nach ihrer Bereitung oder nach einer Standzeit, z. B. nach zeitlich unbegrenzter Lagerung, eingesetzt, z. B.
- für weitere (z.B. NH₂-reaktive) Funktionalisierungen mit hydrophoben, hydrophilen, geladenen, redoxaktiven Strukturen bzw. pH- oder Licht-Absorptions-Eigenschaften und/oder
- für die adhäsive oder kovalente Fixierung von Funktionsmolekülen, z. B. Bio-Funktionsmolekülen, wie Proteinen, DNA-, RNA- oder Protein-Aptameren, Zellen bzw. Zellbestandteilen oder Wirkstoffen.

### Beispiel 1: Si-Verbundmaterial mittels Aminocellulosetosylate

Si-Substratproben wurden in einer 0,05%igen wässrigen Lösung eines Aminocellulosetosylates (Typ b, Formelschema 1), z. B. bei Raumtemperatur
- 5 Min. geschüttelt oder
- 5 Min. im Utraschallbad behandelt oder
- 3 h stehengelassen.

Danach wurden die modifizierten Si-Substratproben 3 bis 4 mal mit jeweils 0,5 bis 1 cm³ bidest. Wasser unter Schütteln, z. B. 3 mal 1 Min. im Ultraschallbad, gewaschen und anschließend im Argon-Strom getrocknet.

Oberflächenstruktur-Charakteristika, z. B. Dickenabmessung (ellipsometrisch) <1 - 3 nm; KW: 60 - 80°; RMS-Rauhigkeit: 0,8 - 2 nm; NH₂-Gruppen-Konzentration: 0,2 - 1,3 nMol/cm².

### Beispiel 2: Si-Verbundmaterial mittels Aminocellulosecarbanilat

Si-Substratproben wurden in einer 0,1%igen Lösung eines Aminocellulosecarbanilates (Typ a, Formelschema 1) in DMA bei Raumtemperatur
- 1 Min. geschüttelt oder
- 5 Min. im Utraschallbad behandelt oder
- 1 h stehengelassen.

Danach wurden die modifizierten Si-Substratproben 3 bis 4 mal mit jeweils 0,5 bis 1 cm³ DMA ca. 5 Min. geschüttelt oder 2 mal im Ultraschallbad gewaschen und anschließend im Argon-Strom getrocknet.

Oberflächenstruktur-Charakteristika, z.B. Dickenabmessung (ellipsometrisch)< 1 - 2 nm; KW: 55 - 70°; RMS-Rauhigkeit: 0,2 - 0,5nm; NH₂-Gruppen-Konzentration: 0,2 - 1,3 nMol/cm².

### Beispiel 3: Si-Verbundmaterial mittels EDA-Cellulose durch spin-coating

Si-Substratproben (D= 10 mm) wurden mittels spin-coating (bei 20 Tausend U/Min.) mit 1 oder 2 *µ*l einer 0,5%igen EDA-Cellulosecarbanilat-Lösung (Typ a, i=2) in DMA bei Raumtemperatur betropft (Rotationsdauer 3 - 5 Min.). Danach wurden die modifizierten Si-Substratproben 2 mal im Ultraschallbad (Zeitdauer ca. 10 Min.) mit jeweils ca. 1 cm³ DMA gewaschen und anschließend im Argon-Strom getrocknet.

Oberflächenstruktur-Charakteristika, z.B. Dickenabmessung (ellipsometrisch) <1 bis 2 nm; Wasser-Kontaktwinkel KW: 60 bis 75°; RMS-Rauhigkeit: < 1 bis 2 nm; NH₂-Gruppen-Konzentration: 0,2 bis 1 nMol/cm².

### Beispiel 4: Si-Verbundmaterial mittels NH₂-(Organo)polysiloxanderivat

Si-Substratproben wurden vorzugsweise in einer 0,05 bis 0,09 %igen Lösung eines NH₂-(Organo)polysiloxanderivates (P1 bis P5, Formelschema 2), z. B. in Ethanol oder 2-Propanol, bei Raumtemperatur
- 1 - 5 Min. geschüttelt oder
- 15 Min. im Utraschallbad behandelt oder
- 6 h stehengelassen.

Danach wurden die modifizierten Si-Substratproben 3 bis 4 mal mit jeweils 300 bis 500 *µ*l Ethanol oder 2-Propanol geschüttelt oder 2 mal im Ultraschallbad (Zeitdauer ca. 5 bis 15 Min.) gewaschen und anschließend im Argon-Strom getrocknet. Es wurden insbesondere auch Gemische der NH₂-Organo)polysiloxane gemäß Formelschema 2, z. B. P3a und P5, P3a und P3c, P2a und P5, in Ethanol oder 2-Propanol eingesetzt.

Bei Anwendung von spin-coating wurden die Si-Substratproben (D= 10 mm) bei ca. 20 Tausend U/Min. mit 1 bis 2 *µ*l der jeweiligen NH₂-(Organo)polysiloxan-Lösung betropft (Rotationsdauer ca. 3 bis 5 Min.) und anschließend wie oben angegeben weiterbehandelt.
Die Oberflächenstruktur-Charakteristika waren in Abhängigkeit vom NH₂-(Organo)polysiloxanderivat von der Konzentration der Derivat-Lösung und von den Bedingungen der unterschiedlichen Prozeduren variierbar.
Oberflächenstruktur-Charakteristika: Dickenabmessungen (ellipsometrisch) < 1 bis 3,5 nm; Wasser-Kontaktwinkel KW: 45 bis 70°; RMS-Rauhigkeiten: 0,5 bis 1,7 nm (maximierbar) und NH₂-Gruppen-Konzentrationen 0,1 bis 2 nMol/cm².

### Beispiel 5: Redoxaktives Si-Verbundmaterial mittels PDA-Cellulosetosylat

Si-Substratproben wurden mit SiOₓ-Polymer behandelt und danach in einer 0,1%igen Lösung eines PDA-Cellulosetosylates (Typ c, 1, Formelschema 1) in DMA, z. B. bei Raumtemperatur
- 30 Minuten geschüttelt oder
- 15 Minuten im Utraschallbad behandelt oder
- 3 h stehengelassen.

Danach wurden die modifizierten Si-Substratproben 3 bis 4 mal mit jeweils 0,5 bis 1 cm³ DMA durch Schütteln gewaschen und anschließend im Argon-Strom getrocknet.

Oberflächenstruktur-Charakteristika: Dickenabmessung (ellipsometrisch) < 1 bis 1,5 nm; Wasser-Kontaktwinkel KW: 70 bis 80°; RMS-Rauhigkeit: < 1 bis 1,5 nm; NH₂-Gruppen-Konzentration: 0,4 bis 1 nMol/cm².

### Beispiel 6: Glas-Verbundmaterial mittels TETAT-Cellulosetosylat

(a) MD-Substratproben, wie genannt vorbehandelt, wurden in einer 0,5%igen wässrigen Lösung eines TETAT-Cellulosetosylates bei Raumtemperatur 30 Min. geschüttelt, danach 3 bis 4 Mal mit jeweils 0,5 bis 1 cm³ bidest. Wasser durch Schütteln oder 2 Mal im Ultraschallbad gewaschen und anschließend im Argon-Strom getrocknet.
   Oberflächenstruktur-Charakteristika: Dickenabmessung < 3 nm; Wasser-Kontaktwinkel KW: 60 bis 75°; RMS-Rauhigheit: < 1nm NH₂-Gruppen-Konzentration: 2 bis 4 nMol/cm².
(b) MD-Substratproben wurden mit SiOₓ-Polymer behandelt und danach in einer 0,05%igen wässrigen Lösung eines TETAT-Cellulosetosylates bei Raumtemperatur 1 Min. geschüttelt, danach 3 bis 4 Mal mit jeweils 0,5 bis 1 cm³ bidest. Wasser durch Schütteln gewaschen und anschließend im Argon-Strom getrocknet.
   Oberflächenstruktur-Charakteristika: Dickenabmessung < 3 nm; Wasser-Kontaktwinkel KW: 60 bis 75°; RMS-Rauhigheit: < 1,5 bis 2,5 nm NH₂-Gruppen-Konzentration: 4,8 bis 5,5 nMol/cm².

### Beispiel 7: Al-Oxid-Verbundmaterial mittels Aminocellulosederivat

Al-Oxid-Substratproben wurden in einer 0,05 bis 5%igen Lösung eines Aminocellulosenderivates (z. B. Typ b, Formelschema 1) z. B. bei Raumtemperatur
- 5 Minuten geschüttelt oder
- 1 bis 2 h stehengelassen.

Danach wurden die modifizierten Al-Oxid-Substratproben 3 bis 4 mal mit jeweils 0,5 bis 1 cm³ des eingesetzten Lösungsmittels (DMA oder bidest. Wasser) durch Schütteln gewaschen und anschließend im Argon-Strom getrocknet.

Oberflächenstruktur-Charakteristika: Dickenabmessung (ellipsometrisch) < 1,5 bis 3 nm; Wasser-Kontaktwinkel KW: 65 bis 80°; RMS-Rauhigkeit: 0,5 bis 1nm; NH₂-Gruppen-Konzentration: 0,5 bis 1 nMol/cm² (z. B. mittels EDA-Cellulosetosylat modifiziert). Oder aber Dickenabmessung (ellipsometrisch) < 1 bis 2 nm; Wasser-Kontaktwinkel KW: 60 bis 75°; RMS-Rauhigkeit: 0,4 bis 1 nm; NH₂-Gruppen-Konzentration: 0,3 bis 0,5 nMol/cm² (mittels EDA-Cellulosecarbanilat modifiziert).

### Beispiel 8: Al-Oxid-Verbundmaterial mittels NH₂-(Organo)polysiloxanderivat

Al-Oxid-Substratproben wurden in einer 0,04 bis 0,1%igen Lösung eines NH₂-(Organo)polysiloxanderivates (z.B. P4b bzw. P5b, Formelschema 2), z. B. in Ethanol oder 2-Propanol, bei Raumtemperatur
- 5 Minuten geschüttelt oder
- 3 h stehengelassen oder
- 30 Minuten im Ultraschallbad behandelt.

Danach wurden die modifizierten Al-Oxid-Substratproben 3 bis 4 mal mit jeweils 0,5 bis 1 cm³ 2-Propanol durch Schütteln gewaschen und anschließend im Argon-Strom getrocknet.

Oberflächenstruktur-Charakteristika: Dickenabmessung (ellipsometrisch)< 1 bis 3 nm; KW: 65 bis 80°; RMS-Rauhigkeit: 0,24 bis 0,5 nm; NH₂-Gruppen-Konzentration: 0,5 bis 1,2 nMol/cm²_{.}

### Beispiel 9: Au-Verbundmaterial via Aminocellulosecarbanilat

Au-Substratproben wurden in einer 0,05 bis 0,1%igen Lösung eines Aminocellulosecarbanilates (Typ a bzw. b, Formelschema 1)in DMA bei Raumtemperatur
- 15 Minuten geschüttelt oder
- 15 Minuten im Utraschallbad behandelt oder
- 3 h stehengelassen.

Danach wurden die modifizierten Au-Substratproben 3 bis 4 mal mit jeweils 0,5 bis 1 cm³ DMA unter Schütteln gewaschen und anschließend im Argon-Strom getrocknet.

Oberflächenstruktur-Charakteristika: Dickenabmessung (ellipsometrisch): 0,5 bis 1 nm; Wasser-Kontaktwinkel KW: 65 bis 70°; RMS-Rauhigkeit: 0,3 bis 0,5 nm; NH₂-Gruppen-Konzentration: 0,2 bis 1,3 nMol/cm².

### Beispiel 10: PDMS-Verbundmaterial via Aminocellulosederivat

PDMS-Substratproben wurden mit Sauerstoff-Plasma behandelt und anschließend in einer 0,05 bis 0,1%iger Lösung eines Aminocellulosederivates (Typ a bzw. b, Formelschema 1) bei Raumtemperatur
- 10 Minuten geschüttelt oder
- 3 h stehengelassen.

Danach wurden die modifizierten PDMS-Substratproben 3 bis 4 mal mit jeweils 0,5 bis 1 cm³ des jeweils eingesetzten Lösungsmittels (DMA oder bidest. Wasser) durch Schütteln gewaschen und anschließend im Argon-Strom getrocknet.

Oberflächenstruktur-Charakteristika: Dickenabmessung (ellipsometrisch) < 2 bis 3 nm; KW: 65 bis 80°; RMS-Rauhigkeit: < 0,7 nm; NH₂-Gruppen-Konzentration: 0,8 bis 1,2nMol/cm² (z.B. via EDA-Cellulosetosylat, Typ a, i=2, modifiziert) oder z.B. Dickenabmessung (ellipsometrisch) < 1 bis 2 nm; RMS-Rauhigkeit: 0,3 bis 0,6 nm; NH₂-Gruppen-Konzentration: 0,5 bis 1 nmol/cm² (z.B. EDA-Cellulosecarbanilat-modifiziert).

### Beispiel 11: Si-Verbundmaterial durch µCP

(a) Si-Substratproben wurden anhand eines PDMS-Stempels mit TETAT-Cellulosetosylat-Strukturmustern in periodisch unterschiedlichen Linienabständen: 2 *µ*m, 1*µ*m, 500 nm und 200 nm und unterschiedlichen Linienbreiten: 2 *µ*m, 1*µ*m, 500 nm und 200 nm gestempelt. Dazu wurde der PDMS-Stempel mit 5 bis 10 *µ*l einer 0,05 bis 0,5%igen wässrigen Lösung eines TETAT-Cellulosetosylates betropft. Anschließend wurde der Stempel mit der benetzten Seite 1 bis 5 sec vorsichtig auf ein Filterpapier gedrückt und danach sofort für 2 bis 15 Minuten mit der Si-Substratproben-Oberfläche durch leichtes Aufdrücken in Kontakt gebracht.
   Danach wurde der Stempel von der Si-Oberfläche entfernt, die gestempelte Si-Substratprobe 15 bis 30 Minuten in bidest. Wasser unter Wechseln der wässrigen Phase geschüttelt und anschließend im Argon-Strom getrocknet.
   Oberflächenstruktur-Charakteristika: (Linien-) Dickenabmessung (ellipsometrisch) < 1 - 2 nm. Bei der AFM-Vermessung wurde das periodische Linienmuster mit den unterschiedlichen Abständen und Linienbreiten verifiziert. Für die Linienbreiten wurden ca. Sollwerte vermessen und (Linien-) Dickenabmessungen < 1 bis 3 nm gefunden.
(b) Bei einer anderen Ausführungsvariante wurde ausgehend vom gleichen PDMS-Stempel, der Si-Substratprobe und einer 0,05 bis 0,5%igen wässrigen Lösung eines TETAT-Cellulosetosylates eine Stempel-Prozedurvariante angewendet. Hierfür wurde ein PDMS-Stempelkissen (Abmessung ca. 1x1 cm, Dicke ca. 3 mm) aus dem "PDMS-Sylgard 184"-Material gegossen und mit der TETAT-Cellulosetosylat-Lösung ca. 3 h unter Rühren getränkt. Der PDMS-Stempel wurde auf das vorbehandelte Stempelkissen gedrückt und anschließend mit der Si-Substratproben-Oberfläche ca. 5 Minuten in Kontakt gebracht. Die gestempelte Substratprobe wurde dann, wie unter (a) beschrieben, behandelt und charakterisiert. Dabei wurden die Ergebnisse der Stempel-Prozedur, wie unter (a) beschrieben, bestätigt.

### Beispiel 12: Oberflächen-Modifizierung von SAW-(Sensor)chips im mikrofluidischen Sensorsystem

Bei der Modifizierung der SAW-Chips wurde von unterschiedlichen SAW-Chipoberflächen ausgegangen, z. B.
(a) SiO₂-Polymeroberfläch als Signal-führende Schicht oder
(b) Au-Oberfläche auf SiO₂-Polymer als Signal-führende Schicht.

Vor Einsetzen des SAW-Chips (b) in das mikrofluidische Sensorsystem wurde die Au-Oberfläche auf die genannten Weisen vorbehandelt.

Schritt 1: Eine 0,5%ige, Lösung eines TETAT-Cellulosetosylates in bidest. Wasser wurde über den SAW-Chip geleitet (Fließgeschwindigkeit ca. 25 *µ*l/Min., Dauer ca. 9 Min.). Das Signal der Phasenänderung, d. h. die Masse-Zunahme, des SAW-Chips war nach ca. 3 Minuten konstant. Danach wurde bidest. Wasser durch das mikrofluidische Sensorsystem geleitet (Fließgeschwindigkeit ca. 25 *µ*l/Min., Dauer ca. 9 Min.), zwecks Ablösung von gegebenenfalls nicht-adhäsiv auf der Chipoberfläche befindlichem TETAT-Cellulosetosylat. Dabei wurde kaum eine Signaländerung, das heißt kaum eine Masse-Ablösung, beobachtet. Der Schritt 1 wurde mit der 0,5%igen TETAT-Cellulosetosylat-Lösung bei gleichen Fließbedingungen wiederholt. Es wurde keine Masse- bzw. Signaländerung des SAW-Chips beobachtet, auch nicht beim anschließenden Durchleiten von bidest. Wasser (Fließbedingungen wie oben). Die Modifizierung der Au-Oberfläche bzw. SiO₂-Polymeroberfläche des SAW-Chips mittels TETAT-Cellulosetosylat war somit innerhalb einer Fließdauer von 3 Minuten beendet.

Schritt 2: Die modifizierte SAW-Chipoberfläche wurde mittels eines NH₂-reaktiven bifunktionellen Reagenz, z. B. Glutaraldehyd (GDA), funktionalisiert. Dazu wurde eine 25%ige wässrige Glutaraldehyd-Lösung über die modifizierte SAW-Chipoberfläche geleitet (Fließgeschwindigkeit ca. 50 *µ*l/Min., Dauer ca. 5 Min.). Anschließend wurde das nicht an der SAW-Chipoberfläche umgesetzte bifunktionelle Reagenz mittels bidest. Wasser bei gleicher Fließgeschwindigkeit und Dauer entfernt. Die vermessene Phasenänderung bzw. Masse-Zunahme signalisiert, dass die SAW-Chipoberfläche via GDA funktionalisiert wurde.

Schritt 3: Ausgehend von der modifizierten SAW-Chipoberfläche wurde z. B. mittels eines Anti-Thrombin-RNA-Aptamers ein Analyt (Thrombin)-sensitiver SAW-Sensorchip hergestellt. Dazu wurde eine Anti-Thrombin-RNA-Aptamer-Lösung in bidest. Wasser (1 *µ*molar) über die SAW-Chipoberfläche geleitet (Fließgeschwindigkeit ca. 25 *µ*l/Min., Dauer ca. 9 Min.). Die resultierende Phasenänderung signalisiert, dass das Aptamer auf der SAW-Chipoberfläche fixiert vorliegt. Bei anschließendem Durchleiten von bidest. Wasser (Fließgeschwindigkeit ca. 25 *µ*l/Min., Dauer ca. 9 Min.) zeigte sich, dass das Aptamer nicht abgelöst wird. Das heißt, der SAW-Sensorchip war geeignet, um Thrombin als Analyt zu vermessen.

Sensor-Test- bzw. Messschritt: Der Test- bzw. Messstatus des mikrofluidischen Sensorsystems wurde durch einen SELEX-Puffer (1 mmolar, pH 8) mit einer Fließgeschwindigkeit von ca. 25 *µ*l/Min. eingestellt. Der SAW-Sensorchip war Thrombinspezifisch und frei von unspezifischer Protein-Bindung, wie sich anhand von Testläufen mittels Thrombin- bzw. Elastase- und Rinderserumalbumin-Lösungen in SELEX-Puffer erwies. Das Thrombin, welches sich nach dem Messschritt auf der Sensoroberfläche befand, wurde mittels 0,1 molarer NaOH-Lösung abgelöst. Die anschließende erneute Vermessung der Thrombin-Lösung in SELEX-Puffer bestätigte, dass der SAW-Sensorchip regenerierbar ist und reproduzierbare Messwerte liefert.

Die Modifizierung von SAW-(Sensor)chips war auch via NH₂-(Organo)polysiloxanderivaten und bei Variation des NH₂-reaktiven Reagenz bzw. des Bio-Funktionsmolekül-Typs erfolgreich.

### Beispiel 13: Funktionalisierung mittels NH₂-reaktiver bifunktioneller Reagenzien

Die Funktionalisierung dient der Modifizierung der genannten Oberflächen-Eigenschaften, insbesondere der Bio-Funktionalisierung.

Allgemeines Verfahren: Zwecks Funktionalisierung wurde das Substratverbundmaterial in einer meist gesättigten Lösung eines NH₂-reaktiven bifunktionellen Reagenz 5 bis 60 Min. geschüttelt oder stehengelassen. Danach wurde die funktionalisierte Substratprobe mehrfach unter Schütteln gewaschen, im Argonstrom getrocknet und für den Anwendungszweck, insbesondere bei der Bio-Funktionalisierung, eingesetzt.

Zur Variation des Wasser-Kontaktwinkels, der pH- oder Ladungsverteilungs-Eigenschaften und/oder zur Bio-Funktionalisierung wurden als bifunktionelle Reagenzien vorzugsweise eingesetzt:
L-Ascorbinsäure, 1,3-Benzol-disulfonylchlorid, 1,4-Benzol-disulfonylchlorid, Phthaldialdehyd, Isophthaldialdehyd, 1,4-Diacetylbenzol, 1,3-Diacetylbenzol, Glutaraldehyd, Benzochinon, 1,3-Benzol-dicarbonsäure dichlorid, 1,4-Benzoldicarbonsäuredichlorid, Cyanurchlorid.

## Patentansprüche

1. Verfahren zur Modifizierung eines Substrates mit den Schritten:
- das Substrat wird mittels einer Modifizierungslösung mit mindestens einem in der Lösung enthaltenen Derivat einer Aminocellulose und/oder
- mit mindestens einem in der Lösung enthaltenen Derivat eines NH₂-(Organo)polysiloxan in Kontakt gebracht,
- es bildet sich ein Substratverbundmaterial aus Substrat und Aminocellulosederivat und/oder Substrat und NH₂-(Organo)polysiloxan aus, wobei ein Glas, Metall, Edelmetall, Metalloxid, Keramik, Silizium, Polysaccharid, Polymer oder Protein umfassendes Substrat gewählt wird.

2. Verfahren nach vorhergehenden Anspruch,
bei dem das modifizierte Substrat mindestens einmal mit einem Lösungsmittel des jeweils eingesetzten Aminocellulosen- bzw. NH₂-(Organo)polysiloxanderivates gewaschen wird.

3. Verfahren nach einem der vorhergehenden Ansprüche,
bei dem das Substratverbundmaterial mittels NH₂-reaktiver und/oder OH-reaktiver Reagenzien gebildet wird.

4. Verfahren nach einem der vorhergehenden Ansprüche,
**dadurch gekennzeichnet, dass**
das Substratverbundmaterial insbesondere mit einer Lösung aus L-Ascorbinsäure, 1,3-Benzol-disulfonylchlorid, 1,4-Benzol-disulfonylchlorid, Phthaldialdehyd, Isophthaldialdehyd, 1,4-Diacetylbenzol, 1,3-Diacetylbenzol, Glutaraldehyd, Benzochinon, 1,3-Benzol-dicarbonsäuredichlorid, 1,4-Benzoldicarbonsäuredichlorid, Cyanurchlorid funktionalisiert bzw. chemisch aktiviert wird.

5. Verfahren nach einem der vorhergehenden Ansprüche,
**dadurch gekennzeichnet, dass**
eine Oberflächenmodifizierung des Substratverbundmaterials mittels bifunktioneller Reagenzien, vorzugsweise NH₂-reaktiver bifunktioneller Reagenzien, und Kopplung von Funktionsmolekülen, vorzugsweise Biofunktionsmolekülen, durchgeführt wird.

6. Verfahren nach einem der vorhergehenden Ansprüche,
bei dem das Substrat zur Ausbildung von OH⁻-Gruppen vor in Kontakt bringen mit der Modifizierungslösung mit einem SiOₓ-Polymer vorbehandelt wird.

7. Verfahren nach vorhergehendem Anspruch,
**gekennzeichnet durch**
Wahl von Proteinen, Nukleinsäure, DNA-, RNA- oder Protein-Aptameren, Zellen bzw. Zellbestandtelle, Antithrombotika oder Wirkstoffe als (Bio-)Funktionsmoleküle.

8. Verfahren nach Anspruch 1,
bei dem das Substrat vor der Modifizierung durch Aminocellulosederivat und/oder NH₂-(Organo)polysiloxanen oxidiert wird.

9. Verfahren nach einem der vorhergehenden Ansprüche,
bei dem als Substrat ein Polysiloxan, insbesondere ein Poly(dimethylpolysiloxan) (PDMS) ausgewählt wird.

10. Verfahren nach einem der vorhergehenden Ansprüche,
mit einer 0,05 bis 0,5 %-igen Modifizierungslösung eines Aminocellulosederivates.

11. Verfahren nach vorhergehendem Anspruch,
**gekennzeichnet durch**
bidestilliertes Wasser oder Dimethylacetamid (DMA) als Lösungsmittel der Modifizierungslösung.

12. Verfahren nach einem der vorhergehenden Ansprüche,
mit einer 0,03 bis 1 %-igen NH₂-(Organo) polysiloxan-Modifizierungslösung, insbesondere einer 0,03 bis 0,1 %-igen Modifizierungslösung.

13. Verfahren nach vorhergehendem Anspruch,
mit Methanol, Ethanol oder 2-Propanol als Lösungsmittel für das NH₂-(Organo)polysiloxan.

14. Verfahren nach einem der vorhergehenden Ansprüche,
**dadurch gekennzeichnet, dass**
das Substrat mit einer 0,05 bis 0,09%igen Lösung eines NH₂-(Organo)polysiloxans der allgemeinen Formel P1b, P2b, P3b, P4b oder P5b gelöst in 2-propanol in Kontakt gebracht wird.

15. Verfahren nach einem der vorhergehenden Ansprüche,
**dadurch gekennzeichnet, dass**
das Substrat zunächst mit einer NH₂-(Organo) polysiloxan-Lösung und sodann mit einer Aminocellulosederivat-Lösung in Kontakt gebracht wird.

16. Verfahren nach vorhergehenden Anspruch,
**gekennzeichnet durch**
ein NH₂-(Organo)polysiloxan der allgemeinen Formel P1b, P2b, P3b, P4b oder P5b des Formelschema 2 und einem Aminocellulosederivat gemäß des Typ a bis c des Formelschema 1.

17. Verfahren nach einem der vorhergehenden Ansprüche,
**dadurch gekennzeichnet, dass**
nach der Modifizierung mit einem NH₂-(Organo)polysiloxanderivat eine Salzsäure-, Schwefelsäure- oder Essigsäurebehandlung erfolgt.

18. Verfahren nach einem der vorhergehenden Ansprüche,
**dadurch gekennzeichnet, dass**
das Substrat mit der Modifizierungslösung 1 Minute bis 6 Stunden, insbesondere 5 Minuten geschüttelt wird.

19. Verfahren nach einem der vorhergehenden Ansprüche,
**dadurch gekennzeichnet, dass**
das Substrat mit der Modifizierungslösung durch Ultraschallbad, spin-coating, dip-coating, air-brush-Verfahren, Mikrokontakt-Drucken (µCP), Schütteln in Kontakt gebracht wird.

20. Substratverbundmatenal hergestellt nach einem der vorhergehenden Ansprüche,
**dadurch gekennzeichnet, dass**
das Aminocellulosederivat und/oder NE₂-(Organo)polysiloxan in Form von Monolagen auf dem Substrat angeordnet ist.

21. Substratverbundmaterial nach vorhergehendem Anspruch,
**gekennzeichnet durch**
eine Dickenabmessung < 5 Nanometer.

22. Substratverbundmaterial nach einem der vorhergehenden Ansprüche 20 oder 21,
**dadurch gekennzeichnet, dass**
das Aminocellulosedezivat und/oder NH₂-(Organo)polysiloxan adhäsiv fest fixiert ist.

23. Substratverbundmaterial nach einem der vorhergehenden Ansprüche 20 bis 22,
**dadurch gekennzeichnet, dass**
die RSM-Rauhigkeit des Substratvex-bundmaterials < 0,2 bis 2 Nanometer beträgt.

24. Verwendung eines Substratverbundmaterials nach einem der vorhergehenden Ansprüche 20 bis 23,
zur Herstellung von Implantaten, Chips, Nanopartikeln oder Rastersondenspitzen.

## Claims

1. Method of modifying a substrate comprising the following steps:
- by means of a modifying solution, the substrate is brought into contact with at least one derivative of an amino cellulose contained in the solution and/or
- with at least one derivative of an NH2-(organo)polysiloxane contained in the solution,
- a composite substrate material is formed from the substrate and the aminocellulose derivative and/or the substrate and NH2-(organo)polysiloxane, wherein a substrate comprising glass, metal, precious metal, metal oxide, ceramic, silicon, polysaccharide, polymer or protein is selected.

2. Method according to the preceding claim, wherein the modified substrate is washed at least once with a solvent of the aminocellulose or NH2-(organo)polysiloxane derivative which is used.

3. Method according to one of the preceding claims, wherein the composite substrate material is formed by means of NH2-reactive and/or OH-reactive reagents.

4. Method according to one of the preceding claims, wherein the composite substrate material is functionalised or chemically activated with a solution of L-ascorbic acid, 1,3-benzene-disulphonylchloride, 1,4-benzene disulphonylchloride, phthaldialdehyde, isophthaldialdehyde, 1,4-diacetylbenzene, 1,3-diacetylbenzene, glutaraldehyde, benzoquinone, 1,3-benzene-dicarboxylic acid dichloride, 1,4-benzenedicarboxylic acid dichloride or cyanurchloride.

5. Method according to one of the preceding claims, **characterised in that** a surface modification of the composite substrate material is performed by means of bifunctional reagents, preferably NH2-reactive bifunctional reagents and by the coupling of functional molecules, preferably bio-functional molecules.

6. Method according to one of the preceding claims, wherein, in order to form OH- groups, the substrate is pretreated with an SiOx polymer before being brought into contact with the modifying solution.

7. Method according to the preceding claim, **characterised by** the selection of proteins, nucleic acids, DNA, RNA or protein aptamers, cells or cell components, antithrombotics or active ingredients as (bio-)functional molecules.

8. Method according to claim 1 wherein the substrate is oxidised prior to modification by the aminocellulose derivative and/or NH2-(organo)polysiloxanes.

9. Method according to one of the preceding claims, wherein a polysiloxane, in particular a poly(dimethylpolysiloxane) (PDMS) is selected as the substrate.

10. Method according to one of the preceding claims, using a 0.05 to 0.5% modifying solution of an aminocellulose derivative.

11. Method according to one of the preceding claims, **characterised by** the use of bidistilled water or dimethylacetamide (DMA) as the solvent of the modifying solution.

12. Method according to one of the preceding claims, using a 0.03 to 1% NH2-(organo)polysiloxane modifying solution, in particular a 0.03 to 0.1% modifying solution.

13. Method according to the preceding claim, wherein methanol, ethanol or 2-propanol are used as the solvent for the NH2-(organo)polysiloxane.

14. Method according to one of the preceding claims, **characterised in that** the substrate is brought into contact with a 0.05 to 0.09% solution of an NH2-(organo)polysiloxane with the general formula P1b, P2b, P3b, P4b or P5b, dissolved in 2-propanol.

15. Method according to one of the preceding claims, **characterised in that** the substrate is first brought into contact with an NH2-(organo)polysiloxane solution and then with an aminocellulose derivative solution.

16. Method according to the preceding claim, **characterised by** an NH2-(organo)polysiloxane with the general formula P1b, P2b, P3b, P4b or P5b of formula schema 2 and an aminocellulose derivative according to types a to c of formula schema 1.

17. Method according to one of the preceding claims, **characterised in that** following modification with an NH2-(organo)polysiloxane derivative, a treatment with hydrochloric acid, sulphuric acid or acetic acid is performed.

18. Method according to one of the preceding claims, **characterised in that** the substrate is agitated with the modifying solution for 1 minute to 6 hours, in particular for 5 minutes.

19. Method according to one of the preceding claims, **characterised in that** the substrate is brought into contact with the modifying solution by means of an ultrasonic bath, spin-coating, dip-coating, air-brushing, micro-contact printing (µCP), or agitation.

20. Substrate compound material produced according to one of the preceding claims, **characterised in that** the aminocellulose derivative and/or NH2-(organo)polysiloxane is arranged on the substrate in the form of monolayers.

21. Composite substrate material according to the preceding claim, **characterised by** a thickness dimension of <5 nanometres.

22. Substrate compound material according to one of the preceding claims 20 or 21, **characterised in that** the aminocellulose derivative and/or NH2-(organo)polysiloxane is fixed in place adhesively.

23. Substrate compound material according to one of the preceding claims 20 to 22, **characterised in that** the RSM roughness of the composite substrate material is <0.2 to 2 nanometres.

24. Use of a composite substrate material according to one of the preceding claims 20 to 23 for the production of implants, chips, nanoparticles or scanning probe tips.

## Revendications

1. Procédé de modification d'un substrat, comportant les étapes suivantes :
- le substrat est mis en contact au moyen d'une solution modifiante avec au moins un dérivé d'une aminocellulose contenu dans la solution et/ou
- avec au moins un dérivé d'un NH₂-(organo)polysiloxane contenu dans la solution,
- il se forme un matériau composite de substrat et un dérivé d'aminocellulose et/ou de substrat et de NH₂-(organo)polysiloxane, un substrat comprenant le verre, un métal, un métal noble, un oxyde métallique, une céramique, le silicium, un polysaccharide, un polymère ou une protéine étant choisi.

2. Procédé selon la revendication précédente,
dans lequel le substrat modifié est lavé au moins une fois avec une solution de dérivé respectif d'aminocellulose ou de NH₂-(organo)polysiloxane utilisé.

3. Procédé selon l'une des revendications précédentes,
dans lequel le matériau composite de substrat est formé avec des réactifs réagissant à NH₂ et/ou réagissant à OH.

4. Procédé selon l'une des revendications précédentes, **caractérisé en ce que**
le matériau composite de substrat est fonctionnalisé ou activé chimiquement en particulier avec une solution d'acide L-ascorbique, de chlorure de 1,3-benzène-disulfonyle, de chlorure de 1,4-benzène-disulfonyle, de phtaldialdéhyde, d'isophtaldialdéhyde, de 1,4-diacétylbenzène, de 1,3-diacétylbenzène, de glutaraldéhyde, de bensoquinone, de dichlorure d'acide 1,3-benzène-dicarboxylique, de dichlorure d'acide 1,4-benzène-dicarboxylique, de chlorure de cyanure.

5. Procédé selon l'une des revendications précédentes, **caractérisé en ce que**
une modification superficielle du matériau composite de substrat est réalisée au moyen de réactifs bifonctionnels, de préférence, des réactifs bifonctionnels réagissant à NH₂ et par couplage de molécules fonctionnelles, de préférence, de molécules bifonctionnelles.

6. Procédé selon l'une des revendications précédentes,
dans lequel le substrat est prétraité pour former des groupes OH avant la mise en contact avec la solution modifiante avec un polymère SiOₓ.

7. Procédé selon la revendication précédente, **caractérisé par**
le choix de protéines, d'acides nucléiques, d'aptamères d'ADN, d'ARN ou de protéines, de cellules ou de composants cellulaires, d'antithrombotiques ou de substances actives en tant que molécules (bio)fonctionnelles.

8. Procédé selon la revendication 1,
dans lequel le substrat est oxydé avant la modification par un dérivé d'aminocellulose et/ou de NH₂-(organo)polysiloxane.

9. Procédé selon l'une des revendications précédentes,
dans lequel on choisit comme substrat un polysiloxane, en particulier un poly(diméthylpolysiloxane) (PDMS).

10. Procédé selon l'une des revendications précédentes,
avec une solution modifiante de 0,05 à 0,5 % d'un dérivé d'aminocellulose.

11. Procédé selon la revendication précédente, **caractérisé par**
de l'eau bidistillée ou du diméthylacétamide (DMA) comme solvant de la solution modifiante.

12. Procédé selon l'une des revendications précédentes,
avec une solution modifiante de 0,03 à 1 % de NH₂-(organo)polysiloxane, en particulier une solution modifiante de 0,03 à 0,1 %.

13. Procédé selon la revendication précédente, avec du méthanol, de l'éthanol ou du 2-propanol comme solvant du NH₂-(organo)polysiloxane.

14. Procédé selon l'une des revendications précédentes,
**caractérisé en ce que**
le substrat est mis en contact avec une solution de 0,05 à 0,09 % d'un NH₂-(organo)polysiloxane de formule générale P1b, P2b, P3b, P4b ou P5b dissous dans du 2-propanol.

15. Procédé selon l'une des revendications précédentes, **caractérisé en ce que**
le substrat est mis en contact tout d'abord avec une solution de NH₂-(organo)polysiloxane et ensuite, avec un dérivé d'aminocellulose.

16. Procédé selon la revendication précédente, **caractérisé par**
un NH₂-(organo)polysiloxane de formule générale P1b, P2b, P3b, P4b ou P5b du schéma de formule 2 et un dérivé d'aminocellulose selon le type a à c du schéma de formule 1.

17. Procédé selon l'une des revendications précédentes, **caractérisé en ce que**
après la modification avec un dérivé de NH₂-(organo)polysiloxane s'effectue un traitement à l'acide chlorhydrique, à l'acide sulfurique ou à l'acide acétique.

18. Procédé selon l'une des revendications précédentes, **caractérisé en ce que** le substrat est basculé avec la solution modifiante pendant 1 minute à 6 heures, en particulier pendant 5 minutes.

19. Procédé selon l'une des revendications précédentes, **caractérisé en ce que**
le substrat est mis en contact avec la solution modifiante par un bain à ultrasons, par revêtement par centrifugation, trempage, air comprimé, impression par microcontact (µCP), basculement.

20. Matériau composite de substrat fabriqué selon l'une des revendications précédentes, **caractérisé en ce que**
le dérivé d'aminocellulose et/ou de NH₂-(organo)polysiloxane est disposé sur le substrat sous la forme de monocouches.

21. Matériau composite de substrat selon la revendication précédente, **caractérisé par**
une épaisseur < 5 nanomètres.

22. Matériau composite de substrat selon l'une des revendications 20 ou 21, **caractérisé en ce que**
le dérivé d'aminocellulose et/ou de NH₂-(organo)polysiloxane est fixé solidement par adhérence.

23. Matériau composite de substrat selon l'une des revendications 20 à 22 précédentes, **caractérisé en ce que**
la rugosité RSM du matériau composite de substrat est < 0,2 à 2 nanomètres.

24. Utilisation d'un matériau composite de substrat selon l'une des revendications 20 à 23 précédentes,
pour la production d'implants, de puces, de nanoparticules ou de pointes de sondes à balayage.
